# EUROPEAN PATENT APPLICATION

(11) **EP 2 692 732 A1**
(43) Date of publication of application: **05.02.2014**
(21) Application number: 12305972.7
(22) Date of filing: 03.08.2012
(51) Int. Cl.: C07K 14/415, A61K 39/36

(54) **Novel allergen from ragweed pollen and uses thereof**

(71) Applicant: Stallergenes S.A., 92183 Antony Cedex (FR)
(72) Inventor: Bordas, Véronique, 92160 Antony (FR); Bussières, Laetitia, 78000 Versailles (FR); Nony, Emmanuel, 92160 Antony (FR); Batard, Thierry, 78000 Versailles (FR); Chabre, Henry, 75014 Paris (FR); Moingeon, Philippe, 91370 Verrières le Buisson (FR); Bouley, Julien, 92120 Montrouge (FR); Lemignon, Maxime, 91100 Corbeil-Essone (FR)
(74) Representative: Blot, Philippe Robert Emile

(57) **Abstract**

The present invention notably concerns a novel major allergen from ragweed pollen, named Amb a X, as well as isoallergens and isoforms thereof. Fragments of the aforementioned polypeptides and homologous polypeptides, in particular homologous polypeptides in related plant species, also make part of the invention. The invention also concerns uses of said polypeptides, in particular for diagnosing and preventing or treating an allergy.

## Description

The present invention notably concerns a novel protein which is a major allergen from ragweed pollen, named Amb a X, as well as isoallergens and isoforms thereof. Fragments of the aforementioned proteins, polypeptides and homologous polypeptides, in particular homologous proteins and polypeptides in related plant species, also make part of the invention. The invention also concerns antibodies against said polypeptides as well as uses of said proteins, polypeptides and antibodies, in particular for diagnosing, preventing or treating an allergy.

Weeds, and in particular weeds belonging to the genera *Ambrosia, Artemisia,* and *Parietaria,* are often associated with pollinosis in the population exposed thereto.

Ragweed denotes a plant of the genus *Ambrosia.* Among plants of this genus, the main sources of pollinosis are Short ragweed, i.e. *Ambrosia artemisiifolia,* which is also called *Ambrosia elatior,* Western ragweed, i.e. *Ambrosia psilostachya,* and Giant ragweed, i.e. *Ambrosia trifida.* In particular, pollen of short ragweed *(Ambrosia artemisiifolia)* is clinically the most important source of seasonal aeroallergens in North America, as it is responsible for the majority and most severe cases of hay fever (allergic rhinitis). Because of the increased spreading of this plant, as well as the increased duration of pollination, there has been considerably more human exposure to short ragweed pollen and thus, the reported cases of hay fever due to short ragweed have risen dramatically.

Plants from the genera *Ambrosia, Artemisia,* and *Parietaria* constitute a homologous group of allergen sources as their allergens display sequence homology and/or cross-reactivity (Lorenz et al., Int. Arch. Allergy Immunol. 2009; 148: 1-17).

So far, allergens of *Ambrosia artemisiifolia* pollen which have been identified include Amb a 1 and Amb a 2, which are major allergens, as well as Amb a 3, Amb a 4, Amb a 5, Amb a 6, Amb a 7, Amb a 8, Amb a 9, and Amb a 10. *Ambrosia psilostachya* pollen notably contains the major allergen Amb p 5, and *Ambrosia trifida* pollen notably contains the major allergen Amb t 5.

Allergens are named using the systematic nomenclature of the Allergen Nomenclature Sub-Committee of the World Health Organization and International Union of Immunological Societies which was revised in 1994 (WHO Bulletin Vol. 72. August 1994; King et al., Allergen nomenclature. Allergy 1995;9:765-74).

In the systematic nomenclature, allergens are designated according to the accepted taxonomic name of their source. Allergen nomenclature comprises three letters, followed by one letter and an Arabic number, and a space after each of the first two elements. The three letters correspond to the first three letters of the genus, the single letter corresponds to the first letter of the species and the numbers are assigned to the allergens in the order of their identification. Thus, an allergen of the short ragweed *Ambrosia artemisiifolia* is designated "Amb a" followed by the number corresponding to the order of its identification.

The same number is generally used to designate homologous allergens of related species. Therefore, allergens designated with a same number, such as Amb a 5, Amb p 5 and Amb t 5 form a "group" of related allergens from different species. For instance Amb a 5, Amb p 5 and Amb t 5 belong to the *Ambrosia* allergen group V.

Furthermore, an allergen from a single species may consist of several closely similar molecules. These similar molecules are designated as "isoallergens" when they share the following common biochemical properties: (a) similar molecular size; (b) identical biological function, e.g. enzymatic action; and (c) ≥67% identity of amino acid sequences (King et al., Allergy 1995, 50:765-774).

Besides, cDNA cloning of allergens often show nucleotide mutations which are either silent or which can lead to single or multiple amino acid substitutions. Therefore, each isoallergen may have multiple forms of closely similar sequences, which are designated as "isoforms". Furthermore, Amb a X and its isoallergens may present different glycosylation patterns.

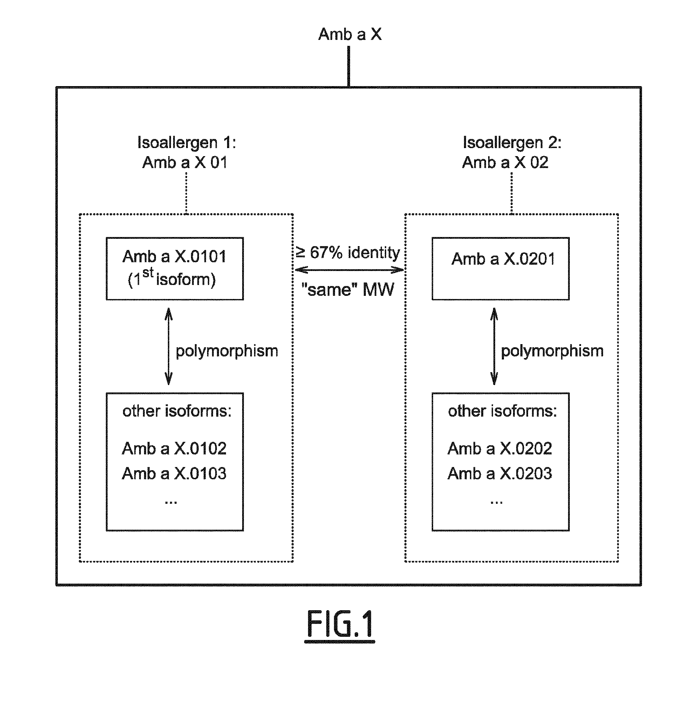

The principles of the allergen nomenclature are illustrated in Figure 1. Accordingly, a newly identified allergen member of a new family of homologous allergens of a same species constitutes the first isoform of the first isoallergen of the family of homologous allergens.

The inventors have identified a new protein which is a major pollen allergen from ragweed *(Ambrosia artemisiifolia).* This protein constitutes the first isoform of the first isoallergen of a new family of homologous allergens called "Amb a X".

Identification of new allergens is essential, as it allows both for diagnosing and characterizing allergies, and for treating said allergies by allergen-specific immunotherapies. Identification of major allergens, i.e. allergens for which more than 50% of patients tested have corresponding allergen-specific IgE, is even more crucial in order to diagnose and treat well-spread allergies. Allergen specific immunotherapy (SIT), or desensitization, is a form of immunotherapy for allergic disorders in which the patient is administered with an allergen preparation with the aim of inducing immunologic tolerance. The sublingual route has been recently explored in the field of SIT. Allergen-specific sublingual immunotherapy (SLIT) indeed represents a safe and efficient non invasive alternative to subcutaneous immunotherapy (SCIT).

### DESCRIPTION OF THE INVENTION

The inventors have performed a study on allergenic sensitization profile of ragweed pollen-allergic patients. They have analysed the allergenic profile by locating the most frequently recognized allergens for 28 patient sera on a synthetic map and have identified them by mass spectrometry. This analysis has led the inventors to identify a new 30-35 kDa ragweed protein reactive with IgE from ragweed allergic patients, which they have named Amb a X, with X designating the still undetermined number corresponding to the order of the identification of this new allergen. Furthermore, the inventors have subsequently characterized this allergen. In particular, they have shown that Amb a X belongs to the cysteine protease family.

Amb a X, which is not recorded in protein and allergen databases, is reactive with 54 % of ragweed allergic patients' sera and is therefore a major allergen. The inventors have thus isolated a novel major allergen from ragweed pollen.

As shown in Example 2, the inventors have cloned by a RACE approach the newly identified ragweed allergen Amb a X using DNA primers designed with peptide sequences identified by 2D electrophoresis followed by mass spectrometry and Edman N-terminal sequencing. Based on the different Amb a X fragments, they have established the following consensus sequence:

M1EINKLVCFSFSLVLILGLVES22F23HYHERELESEEGFMGMYDRWREQHNIEMRSPERF
NVFKYNVRRIHESNKMDKPYKLKVNEFADMTNLEFVNTYANSKISHFQALR108G109SAPG
SIDTDPNKDFIYANVTKIPDKVDWREKNAVTDVKGQGGCGSCWAFAAVVALEGINAIRT
GKLVKFSEQQLVDCDMTNAGCDGGLMEPAFTYVIKHGGIAPEASYPYVGKRETCDKA
KIKDVLKIDGRQNVPGLDEEALRKAVAHQPVATGIQLSGHGLQFYSEGVYTGDCGTEP
NHGVGIVGYGENEKGIKFWTVKNSWGPTWGEKGYIHLQRGARKEGLCGVAMHSSFPI
MNDPNPPKDDPNGPKDDPDAPKDPKFKTTQRLQGIRTKLLEL386 (SEQ ID NO:28).

This sequence SEQ ID NO:28 represents the pre-pro-sequence of Amb a X, in which amino acids at positions 1 to 22 identify a putative signal sequence, amino acids at positions 23 to 108 identify the pro-region, and amino acids at positions 109 to 386 identify the sequence of the mature polypeptide.

By "consensus sequence" it is intended the sequence defined by the most frequent amino acid or nucleotide at each position, in an alignment of available complete protein coding sequences or nucleic acid sequence.

Thus, a consensus sequence of the mature polypeptide Amb a X is:

GSAPGSIDTDPNKDFIYANVTKIPDKVDWREKNAVTDVKGQGGCGSCWAFAAVVALEGI
NAIRTGKLVKFSEQQLVDCDMTNAGCDGGLMEPAFTYVIKHGGIAPEASYPYVGKRETC

DKAKIKDVLKIDGRQNVPGLDEEALRKAVAHQPVATGIQLSGHGLQFYSEGVYTGDCGT
EPNHGVGIVGYGENEKGIKFWTVKNSWGPTWGEKGYIHLQRGARKEGLCGVAMHSSFP
IMNDPNPPKDDPNGPKDDPDAPKDPKFKTTQRLQGIRTKLLEL (SEQ ID NO: 1).

A nucleic sequence encoding the pre-pro-sequence of ragweed allergen Amb a X is given below:

ATGGAAATCAACAAGTTAGTTTGTTTTTCATTTTCTTTGGTTTTGATTTTAGGACTTGTA
GAGAGCTTCCATTACCATGAGAGAGAGCTCGAATCGGAGGAGGGGTTTATGGGGAT
GTATGATAGATGGAGGGAGCAACACAATATCGAAATGAGAAGCCCGGAACGGTTCAA
TGTGTTCAAGTACAATGTTAGGCGCATTCACGAATCGAATAAGATGGACAAGCCGTA
TAAGTTGAAGGTGAACGAGTTTGCTGACATGACTAACCTTGAGTTCGTTAACACGTAT
GCTAACTCGAAGATTAGCCATTTTCAAGCCCTCCGAGGATCAGCACCTGGCTCGATT
GATACCGACCCTAATAAAGATTTCATATATGCAAATGTCACTAAAATCCCAGATAAGG
TCGATTGGAGGGAGAAAAACGCTGTCACTGATGTCAAGGGTCAAGGCGGATGTGGA
AGTTGTTGGGCGTTTGCCGCTGTGGTTGCACTGGAAGGAATAAACGCGATCAGAAC
CGGGAAGCTGGTAAAATTTTCCGAACAACAACTTGTCGATTGTGACATGACGAACGC
AGGATGCGACGGAGGGCTAATGGAACCTGCATTCACATACGTCATAAAGCATGGAG
GTATAGCTCCAGAAGCGAGCTACCCTTACGTAGGCAAAAGAGAAACCTGCGACAAAG
CAAAGATTAAAGATGTGTTGAAGATCGATGGTAGACAAAATGTGCCTGGACTTGACG
AAGAAGCACTAAGGAAGGCAGTTGCACACCAGCCTGTAGCTACCGGTATACAACTTA
GCGGCCATGGTTTGCAGTTCTATTCCGAGGGTGTATATACCGGAGATTGTGGTACAG
AGCCGAATCATGGTGTTGGAATTGTGGGATACGGTGAGAATGAAAAGGGGATTAAAT
TCTGGACCGTGAAGAACTCATGGGGACCAACATGGGGAGAGAAGGGATACATACAT
TTACAACGCGGAGCTAGGAAAGAGGGACTATGCGGAGTAGCAATGCATTCTTCTTTT
CCTATTATGAACGACCCAAACCCACCTAAAGACGACCCCAATGGACCTAAAGACGAC
CCTGATGCACCTAAGGACCCCAAATTTAAAACGACTCAGAGGTTGCAAGGGATAAGG
ACTAAATTGTTGGAGTTGTGA (SEQ ID NO: 29).

### Polypeptides

The invention therefore relates to an isolated protein or polypeptide comprising, or consisting of:
a) the sequence SEQ ID NO: 1 (Amb a X), or
b) a sequence having at least 57% identity with sequence SEQ ID NO: 1 and which has a same biological activity as the polypeptide of sequence SEQ ID NO: 1, or
c) a variant of the sequence defined in a) or b) which exhibits reduced allergenicity or reduced enzymatic activity as compared with the sequence defined in a) or b), or
d) a derivative of the sequence defined in a), b) or c) which has been modified by thermal, chemical or physical treatment, or
e) a fragment of the sequence defined in a), b) or c), said fragment comprising at least 250 contiguous amino acids of the sequence defined in a), b) or c), or being an epitopic fragment of the sequence defined in a), b) or c).

As used hereafter "Amb a X" denotes the mature form of the protein.

The protein or polypeptide comprising, or consisting of, a sequence having at least 57% identity with sequence SEQ ID NO: 1 and which has a same biological activity as the polypeptide of sequence SEQ ID NO: 1 preferably comprises, or consists of, a sequence at least 60%, 67%, 70%, 75%, 80%, 85%, 86%, 87%, 88%, 89%, 90%, 91%, 92%, 93%, 94%, 95%, 96%, 97%, 98%, or 99% identical to the sequence SEQ ID NO: 1 (this polypeptide comprising, or consisting of, a sequence having at least 57% identity with sequence SEQ ID NO: 1 is hereafter referred to as the "homologous polypeptide").

The homologous polypeptide is an "allergen", i.e. a polypeptide which has the capacity to elicit IgEs when administered to a mammal, in particular to a human, sensitised thereto.

By a protein or polypeptide having an amino acid sequence at least, for example, 95% "identical" to the sequence SEQ ID NO: 1, it is intended that the amino acid sequence of the polypeptide, after global pairwise alignment with the sequence SEQ ID NO: 1, the polypeptide sequence may include up to five amino acid modifications per each 100 amino acids of the sequence SEQ ID NO: 1. In other words, to obtain a polypeptide having an amino acid sequence at least 95% identical to the sequence SEQ ID NO: 1, up to 5% (5 of 100) of the amino acid residues in the subject sequence may be inserted, deleted, or substituted with another amino acid.

The percentage of identity between two sequences may be determined by global pairwise alignment using the Needleman-Wunsch algorithm. The percentage of sequence identity can be readily determined for instance using the program Needle, with the BLOSUM62 matrix, and the following parameters gap-open=10, gap-extend=0.5.

The homologous polypeptide of the invention may differ from SEQ ID NO: 1 by one or more modifications, such as i.e. addition, deletion and/or substitution, of one or more amino acids. The protein or polypeptide of the invention may for instance differ from SEQ ID NO: 1 by 1, 2, 3, 4, 5, 6, 7, 8, 9, 10, 12, 15, 20, or 30 amino acids. In particular, the protein or polypeptide of the invention may be a naturally occurring sequence which diverges from the reference sequence SEQ ID NO: 1 by some point mutations, such as e.g. those listed in table 1 (below).

Thus, taking SEQ ID NO: 1 as a reference sequence for the numbering of amino acid positions, mutations are preferably located at one or more of the amino acid positions 97, 104 (which are naturally occurring mutation positions in the mature form of Amb a X), 249, and 252.

Amino acid substitutions may be conservative or non-conservative. Preferably, substitutions are conservative substitutions, in which one amino acid is substituted for another amino acid with similar structural and/or chemical properties, as indicated in table 1.

**Table 1: Description of conservative amino acid substitutions**

| **Conservative substitutions** | **Type of Amino Acid** |
|---|---|
| Ala, Val, Leu, Ile, Met, Pro, Phe, Trp | Amino acids with aliphatic hydrophobic side chains |
| Ser, Tyr, Asn, Gln, Cys | Amino acids with uncharged but polar side chains |
| Asp, Glu | Amino acids with acidic side chains |
| Lys, Arg, His | Amino acids with basic side chains |
| Gly | Neutral side chain |

The homologous protein or polypeptide comprising, or consisting of, a sequence at least 57%, 60%, 67%, 70%, 75%, 80%, 85%, 86%, 87%, 88%, 89%, 90%, 91%, 92%, 93%, 94%, 95%, 96%, 97%, 98%, or 99% identical to the sequence SEQ ID NO: 1, and which has a same biological activity as the polypeptide of sequence SEQ ID NO: 1, preferably differs from the sequence SEQ ID NO: 1 by conservative substitutions only.

The homologous protein or polypeptide preferably comprises, or consists of, a sequence
i) at least 57%, 60%, 67%, 70%, 75%, 80%, 85%, 86%, 87%, 88%, 89%, 90%, 91%, 92%, 93%, 94%, 95%, 96%, 97%, 98%, or 99% identical to the sequence SEQ ID NO: 1, and
ii) which has a same biological activity as the polypeptide of sequence SEQ ID NO: 1, and
iii) which sequence is encoded by the genome of a plant species, in particular a plant species related to *Ambrosia artemisiifolia.* Preferably said plant species related to *Ambrosia artemisiifolia* is another ragweed plant species such as *Ambrosia psilostachya,* or *Ambrosia trifida,* or is a weed, and in particular a weed belonging to the genera *Artemisia* (in particular mugwort or *Artemisia vulgaris)* or *Parietaria* (in particular *Parietaria judaica* or *Parietaria officinalis).*

By "encoded by the genome of a plant species" it is meant that the plant species has not been genetically modified or engineered (by inserting a transgene) to express the homologous protein or polypeptide of the invention.

Said homologous protein or polypeptide is an allergen belonging to the same group of allergens to which Amb a X belongs.

In some embodiments, the homologous protein or polypeptide according to the invention comprises of consists of a sequence which differs from SEQ ID NO: 1 by at least the following mutations:
a) I instead of V in position 97 of SEQ ID NO:1 (position 205 of SEQ ID NO:28); or
b) V instead of A in position 104 of SEQ ID NO:1 (position 212 of SEQ ID NO:28); or
c) A instead of G in position 249 of SEQ ID NO:1 (position 357 of SEQ ID NO:28); or
d) V instead of G in position 249 of SEQ ID NO:1 (position 357 of SEQ ID NO:28); or
e) E instead of D in position 252 of SEQ ID NO:1 (position 360 of SEQ ID NO:28); or
f) I instead of V in position 97 of SEQ ID NO:1 (position 205 of SEQ ID NO:28) and V instead of A in position 104 of SEQ ID NO:1 (position 212 of SEQ ID NO:28); or
e) V instead of G in position 249 of SEQ ID NO:1 (position 357 of SEQ ID NO:28) and E instead of D in position 252 of SEQ ID NO:1 (position 360 of SEQ ID NO:28); or
f) I instead of V in position 97 of SEQ ID NO:1 (position 205 of SEQ ID NO:28) and A instead of G in position 249 of SEQ ID NO:1 (position 357 of SEQ ID NO:28); or
e) I instead of V in position 97 of SEQ ID NO:1 (position 205 of SEQ ID NO:28) and V instead of G in position 249 of SEQ ID NO:1 (position 357 of SEQ ID NO:28) and E instead of D in position 252 of SEQ ID NO:1 (position 360 of SEQ ID NO:28).

In the homologous protein or polypeptide, the amino acid modifications as compared with SEQ ID NO: 1 are preferably located at positions such that they do not significantly undermine the biological activity of the polypeptide. Indeed, the polypeptide of the invention having at least 57% identity with SEQ ID NO: 1 exhibits the same biological activity as the polypeptide of sequence SEQ ID NO: 1.

A "same biological activity" may denote a same biological function. Therefore, in the context of the invention, a polypeptide having a same biological activity as the polypeptide of sequence SEQ ID NO: 1 may for instance be a polypeptide exhibiting the same protease function, preferably the same cysteine protease function. The activity of a compound can easily be evaluated in vitro or in vivo by the person skilled in the art, particularly by means of the following tests: zymogram or analysis of synthetic labelled substrate cleavage in the presence and in the absence of a cysteine protease specific inhibitor, such as E-64.

Alternatively, a polypeptide having a "same biological activity" as the polypeptide of sequence SEQ ID NO: 1" may refer to a polypeptide exhibiting a "same allergenicity", i.e. a polypeptide exhibiting cross-reactivity with IgE antibodies binding to the sequence SEQ ID NO: 1. In particular, a polypeptide having the same allergenicity as Amb a X requires that the polypeptide comprises one or more IgE epitopes identical to those contained in the polypeptide of sequence SEQ ID NO: 1. IgE epitopes contained in the polypeptide of sequence SEQ ID NO: 1 may be identified by methods such as peptide arrays ELISA inhibition, X-ray crystallography, NMR, or hydrogen/deuterium exchange mass spectrometry, using Amb a X specific IgEs, for instance obtained from ragweed allergic patients'sera.

A homologous polypeptide may be in particular a protein which is an isoallergen of sequence SEQ ID NO: 1.

Pursuant to the nomenclature for allergens (King et al. Allergy 1995, 50:765-774), two allergens from a single species are "isoallergens" when a) their sequences have at least 67% sequence identity, b) they have similar molecular weight, and c) they have the same biological function.

As used herein "a polypeptide having similar molecular weight" is intended to denote a polypeptide which theoretical molecular weight differs by about no more than 10% from the molecular weight of a polypeptide consisting of SEQ ID NO: 1. The theoretical molecular weight of a protein or polypeptide is calculated based on the amino acid composition, without taking into account possible glycosylation(s).

The theoretical molecular weight of mature Amb a X (SEQ ID NO: 1) is 30 kDa, and the theoretical molecular weight of the pre-pro-form of Amb a X (SEQ ID NO: 28) is 43 kDa.

Thus, the protein or polypeptide of the invention may in particular comprise or consist of a isoallergen polypeptide having:
(i) at least 67% identity with sequence SEQ ID NO: 1, and in particular at least 70%, 75%, 80%, 85%, 86%, 87%, 88%, 89%, 90%, 91%, 92%, 93%, 94%, 95%, 96%, 97%, 98%, or 99% identity with sequence SEQ ID NO: 1; and
(ii) the molecular weight of said isoallergen polypeptide differs by no more than 10% from the molecular weight of a polypeptide consisting of SEQ ID NO: 1; and
(iii) said isoallergen polypeptide has the same biological activity as the polypeptide of sequence SEQ ID NO: 1.

A homologous protein or polypeptide may also be an allergen isoform of
i) said allergen consisting of sequence SEQ ID NO : 1 (Amb a X), or
ii) an isoallergen of Amb a X.

As used herein, two allergens from a single species are "isoforms" when their sequences have at least 85%, 86%, 87%, 88%, 89%, or preferably at least 90%, 91%, 92%, 93%, 94%, 95%, 96%, 97%, 98%, or 99% identity. Isoforms are naturally occurring allelic or polymorphic variants of an allergen.

In particular, an isoform of the polypeptide of sequence SEQ ID NO: 1 comprises, or consists of, the sequence SEQ ID NO: 1 in which Ile is substituted for Val at position 97 (V>I₉₇).

An isoform of the polypeptide of sequence SEQ ID NO: 1 may also comprise, or consist of, the sequence SEQ ID NO: 1 in which Ile is substituted for Val in position 97 of SEQ ID NO:1, and/or Val is substituted for Ala in position 104 of SEQ ID NO:1, and/or Ala is substituted for Gly in position 249 of SEQ ID NO:1, and/or Val is substituted for Gly in position 249 of SEQ ID NO:1, and/or Glu is substituted for Asp in position 252 of SEQ ID NO:1.

By definition, isoallergens and isoforms of the polypeptide of sequence SEQ ID NO: 1, as well as isoforms of isoallergens of the polypeptide of sequence SEQ ID NO: 1, are *Ambrosia artemisiifolia* pollen allergens.

The polypeptide according to the invention may also be a modified allergen. In particular, the characteristic of the native allergen which has been modified may be its capacity to stimulate the immune system. The capacity to stimulate the immune system may include modifications of T cell epitopes, antibody binding properties, i.e. B cell epitopes, as well as other modifications leading to a modulated capacity to stimulate the immune system.

Therefore, the invention further relates to a "variant polypeptide" comprising, or consisting of a variant of:
a) the sequence SEQ ID NO: 1 or
b) a sequence at least 57% identical with sequence SEQ ID NO: 1 and which has the same biological activity as the polypeptide of sequence SEQ ID NO: 1, as defined above,
said variant polypeptide exhibiting reduced allergenicity as compared with the polypeptide defined in a) or b), as appropriate. This means that said variant polypeptide comprising, or consisting of, a variant of the sequence SEQ ID NO: 1 exhibits reduced allergenicity as compared with the polypeptide of sequence SEQ ID NO: 1. This also means that said variant polypeptide comprising, or consisting of a variant of said sequence defined in b) exhibits reduced allergenicity as compared with the polypeptide of sequence defined in b).

Preferably, the sequence defined in b) is the sequence of an isoallergen of the polypeptide of sequence SEQ ID NO: 1 (Amb a X), or of an isoform of Amb a X, or of an isoform of an isoallergen of Amb a X. Preferably also, the sequence defined in b) is the sequence of an allergen belonging to the same group of allergens to which Amb a X belongs, or of an allergen which is naturally expressed or encoded by the genome of a plant species related to *Ambrosia artemisiifolia,* as described above.

Preferably, said variant polypeptide retains immunogenicity, thus being able to stimulate a B cell-based response essentially without, or without, triggering an IgE-based allergic response. Such a polypeptide may be useful for the diagnosis and/or therapy of allergy.

In the context of the invention, the expression "reduced allergenicity" means that the variant polypeptide exhibits significantly reduced allergenic activity in an *in vitro* or *in vivo* assay designed to measure such allergenicity. Such assays are well known in the art and include, for example, assay of histamine release from basophils of allergen-sensitive patient(s) or model animal sensitised to the native allergen, following challenge with the variant polypeptide and measure of airway hyper-responsiveness. Allergenicity may also be determined by assaying IgE binding capacity of the variant polypeptide for instance using pooled sera of patients sensitised to the native allergen in a immunoassay such as ELISA or RAST.

The expression "retaining immunogenicity" (in any grammatical form) means that the variant polypeptide elicits a non-IgE immune response which is comparable to the non- IgE immune response elicited by the native allergen. Preferably, the IgGs elicited by the variant polypeptide according to the invention will cross react with epitopes present on the native allergen. This IgG response can block IgE binding, thus reducing or preventing allergic responses to the native allergen. In addition, the variant polypeptide may elicit T cell anergy and other allergy suppressive immune responses.

In particular, the characteristic to be modified may be the antibody binding properties of the polypeptide, such as e.g. the IgE binding properties of the polypeptide. Such a modification may be achieved by using the three-dimensional structure to identify potential IgE antibody binding sites (B-cell epitopes) on the surface of the molecule, and subsequently substituting or modifying one or more amino acids of the identified site to destroy one or more IgE epitope(s).

The three-dimensional structure of a polypeptide may be determined by physical methods that are well known in the art, including X-ray crystallography, NMR spectroscopy and electron crystallography. The three dimensional structure of a polypeptide may also be inferred by comparison to an homologous polypeptide, whose structure has been determined empirically by a physical method, as for example by aligning and comparing amino acid sequences.

Typically, the IgE binding properties of the variant polypeptide may be modified with the purpose of providing hypoallergenic allergens, i.e. polypeptides having a reduced potential for eliciting undesired stimulation of the immune system, in particular anaphylactic reactions. In the context of the invention, the expression "reduced IgE reactivity" can mean that a polypeptide or antigen elicits a significantly reduced IgE-predominated humoral immune response, in comparison to the immune response elicited by the native allergen, as measured for instance in an *in vitro* assay performed on blood or plasma taken from an allergen sensitive patient or an experimental animal following challenge. Such *in vitro* assays are well known in the art and include, for example, histamine release assays or immunoassays such as ELISA or RAST.

For instance, the polypeptide according to the invention may be an allergen hybrid polypeptide having reduced allergenicity but retaining immunogenicity in respect of the native allergen, as described in EP 1499349. Thus, the polypeptide exhibiting reduced IgE reactivity may be an Amb a X allergen hybrid polypeptide comprising an epitopic fragment of the native Amb a X allergen and a scaffold protein that is structurally homologous to the Amb a X allergen polypeptide, wherein the hybrid polypeptide has a native conformation and the peptide epitope sequence is present in a surface accessible region of the hybrid polypeptide corresponding to its position in the native Amb a X allergen.

Alternatively, the polypeptide according to the invention may be a mutant of the naturally occurring Amb a X allergen having at least four substitutions of one surface-exposed amino acid residue with another residue, said substitutions being mutually spaced by at least 15 Å and placed in such a manner that at least one circular surface region with an area of 800 Å² comprises no mutation. Methods for obtaining said mutant are described in EP 1 373 510.

Alternatively or additionally, the polypeptide characteristic to be modified may be its proteolytic activity. Such modification may be achieved by studying the three-dimensional structure of the polypeptide, identifying the active site region of the molecule and changing it by substituting or chemically modifying one or more amino acids of the active site region. In particular, the inventors have shown that Amb a X has a cysteine protease function.

Thus, the invention further relates to a variant polypeptide comprising , or consisting of a variant of:
a) the sequence SEQ ID NO: 1 or
b) a sequence having at least 57% identity with sequence SEQ ID NO: 1, and which has the same biological activity as the polypeptide of sequence SEQ ID NO: 1, as defined above,
said variant polypeptide exhibiting reduced enzymatic activity as compared with the polypeptide defined in a) or b), as appropriate.

In the context of the invention, the expression "reduced enzymatic activity" means that a polypeptide or antigen exhibits significantly reduced proteolytic activity in an *in vitro* assay designed to measure such proteolytic activity. Such *in vitro* assays are well known in the art and include, for example, zymogram or labelled synthetic substrate cleavage assay. In particular, the polypeptide exhibiting reduced enzymatic activity according to the invention may comprise one or more mutated cysteine(s) located in its catalytic site and/or involved in its cysteine protease function.

The catalytic triad of Amb a X is constituted by amino acids C₄₇, H₁₈₁, N₂₀₂ of SEQ ID NO: 1. Thus, preferably a variant polypeptide exhibiting reduced enzymatic activity, as compared with the polypeptide of sequence SEQ ID NO: 1, is obtained by modifying at least one amino acid at position 47, 181 or 202 of SEQ ID NO: 1.

The variant polypeptide as disclosed herein preferably exhibits reduced allergenicity and reduced enzymatic activity.
The polypeptide according to the invention may also be an allergen derivative, such as e.g. an "allergoid". Said allergoid may be generated by incubating the protein or polypeptide of the invention in the presence of cross-linking agents such as e.g. formaldehyde or glutaraldehyde. These chemicals react with primary amino groups on specific amino acid residues in the polypeptide, leading to disruption of both 3D and linear epitopes. Thus, allergoids are preferably presented to T cells by APC and less to B cells in comparison to native allergens. This treatment also leads to intra- and inter-molecular cross-linking, leading to the formation of high-molecular-weight complexes showing a reduced capacity to bind IgE, thereby minimizing the release of anaphylaxis-inducing mediators. Furthermore, the process of generating allergoids positively influences the stability of extracts by reducing the internal enzymatic activity of allergen extracts and decreasing their susceptibility to degradation by formation of highly stable complexes. The allergoid may also be generated by thermal or physical treatment of the protein or polypeptide of the invention, as described in Ferreira et al., Inflamm Allergy Drug Targets. 2006;5:5-14 and Egger et al., Front Biosci. 2009;1:77-90.

Any fragment of the isolated polypeptide comprising or consisting of a) the sequence SEQ ID NO: 1, or b) a sequence having at least 57% identity with sequence SEQ ID NO: 1 and which has a same biological activity as the polypeptide of sequence SEQ ID NO: 1, or c) a variant of the sequence defined in a) or b) which exhibits reduced allergenicity or reduced enzymatic activity as compared with the sequence defined in a) or b), and which comprises at least 250, preferably 255, more preferably 260, contiguous amino acids of the sequence defined in a), b) or c), or which is an epitopic fragment of the sequence defined in a), b) or c), also forms part of the invention.

In the context of the invention, a "fragments" of a given polypeptide sequence refers to a stretch of contiguous amino acids of said polypeptide sequence which is shorter than the complete polypeptide sequence. In particular a fragment may consist of at least 5, 6, 7, 8, 9, 10, 11, 12, 13, 14, 15, 20, 25, 30, 35, or 40 consecutive amino acids of said polypeptide sequence. Preferably, a fragment contains no more than 250, 200, 150, 100, 50, or 25 consecutive amino acids of said polypeptide sequence.

The fragment of the invention may be in particular an epitopic fragment of the polypeptide according to the invention. As used herein, an "epitopic fragment" of a polypeptide denotes a stretch of contiguous amino acids of said polypeptide that is recognized by the immune system, specifically by antibodies, B cells, or T cells, and preferably by IgG or IgE. Epitopes may for instance be mapped using protein microarrays, ELISPOT or ELISA techniques. An "epitopic fragment" according to the invention denotes a stretch of contiguous amino acids that is recognized by an antibody against a polypeptide of the invention. Epitopes are typically peptides between 5 and 40 amino acids in length, preferably between 6 and 15 amino acids in length, even more preferably between 8 and 11 amino acids in length. In particular, an epitopic fragment may comprise, or consist of, a fragment of sequence GSAPGSIDTDPNKDF (SEQ ID NO: 30), in particular comprise, or consist of, a fragment of sequence GSAPGSIDTDPNKDFIYANVTKIPD (SEQ ID NO: 31). An epitopic fragment may also be a predictive epitopic fragment which may be identified with an epitope prediction algorithm. For instance, an epitopic fragment according to the invention may be any predictive epitopic fragment identified with the Syfpeithi algorithm, in particular a fragment of one of the sequences SEQ ID NO: 32 to 41 which have been identified for the MHC II molecules HLA-DRB1 *0101, HLA-DRB1 *0401 or HLA-DRB1 *1501.

Alternatively, the fragment of the invention may be a calibration standard fragment useful as a reference for allergen quantification by mass spectrometry.

By an "isolated" polypeptide, it is intended that the polypeptide is no longer in its natural environment within *Ambrosia artemisiifolia.* When referring to a polypeptide, "purified" means that the indicated molecule is present in the substantial absence of other biological macromolecules of the same type. The term "purified" as used herein preferably means at least 75% by weight, more preferably at least 85% by weight, more preferably still at least 95% by weight, and most preferably at least 98% by weight, of biological macromolecules of the same type are present.

The polypeptide according to the invention may be produced by any well-known procedure in the art. For instance, the polypeptide of the invention may be purified or isolated from natural ragweed pollen raw materials or natural ragweed pollen allergen extracts. Pollen raw materials or pollen allergen extracts may be prepared by any method comprising extracting allergens from ragweed pollen. In particular, pollen raw materials or allergen extracts may be prepared by extracting allergens from pollen with aqueous solution, e.g. with an aqueous hydrogenocarbonate solution, followed by separation, clarification by filtration, and ultrafiltration on a 1-10 kDa membrane with washing with at least 2.5 volumes of purified water, or any other suitable washing buffer.

Allergen extracts thus produced may be further purified, especially to reduce the amount of flavonoids contained in the pollen extracts, which can induce genotoxicity, according to the method described in the patent application WO 2010/139809. Said method comprises an ultrafiltration step of the allergen extract on a 5-10 kDa membrane with at least 5 volumes, preferably 10 to 30 volumes of purified water, optionally containing a buffered solution such as an ammonium bicarbonate solution or a phosphate buffered solution.

An allergenic extract may naturally contain one or more isoforms of the same allergen. In a preferred embodiment the allergen is in the form of an extract.

The polypeptide may also be chemically synthesised, in particular where its length does not exceed e.g. 50 amino acids, or more preferably 40, 30, or 20 amino acids.

Examples of chemical synthesis technologies are solid phase synthesis and liquid phase synthesis. As a solid phase synthesis, for example, the amino acid corresponding to the C-terminus of the peptide to be synthesized is bound to a support which is insoluble in organic solvents, and by alternate repetition of reactions, one wherein amino acids with their amino groups and side chain functional groups protected with appropriate protective groups are condensed one by one in order from the C-terminus to the N- terminus, and one where the amino acids bound to the resin or the protective group of the amino groups of the peptides are released, the peptide chain is thus extended in this manner. Solid phase synthesis methods are largely classified by the tBoc method and the Fmoc method, depending on the type of protective group used. Typically used protective groups include tBoe (t-butoxycarbonyl), Cl-Z (2-chlorobenzyloxycarbonyl), Br-Z (2-bromobenzyloyycarbonyl), Bzl (benzyl), Fmoc (9-fluorenylmcthoxycarbonyl), Mbh (4, 4'-dimethoxydibenzhydryl), Mtr (4-methoxy-2, 3, 6-trimethylbenzenesulphonyl), Trt (trityl), Tos (tosyl), Z (benzyloxycarbonyl) and Clz-Bzl (2, 6-dichlorobenzyl) for the amino groups; NO2 (nitro) and Pmc (2,2, 5,7, 8-pentamethylchromane-6-sulphonyl) for the guanidino groups); and tBu (t-butyl) for the hydroxyl groups). After synthesis of the desired peptide, it is subjected to the de-protection reaction and cut out from the solid support. Such peptide cutting reaction may be carried with hydrogen fluoride or tri-fluoromethane sulfonic acid for the Boc method, and with TFA for the Fmoc method.

The method of producing the peptide may optionally comprise the steps of chemically modifying said peptide, to improve their stability and/or their biodisponibility. Such chemical modifications aim at obtaining peptides with increased protection of the peptides against enzymatic degradation *in vivo,* and/or increased capacity to cross membrane barriers, thus increasing its half-life and maintaining or improving its biological activity. Any chemical modification known in the art can be employed according to the present invention. Such chemical modifications include but are not limited to:
- modifications to the N-terminal and/or C-terminal ends of the peptides such as e.g. N-terminal acylation (preferably acetylation) or desamination, or modification of the C-terminal carboxyl group into an amide or an alcohol group;
- modifications at the amide bond between two amino acids: acylation (preferably acetylation) or alkylation (preferably methylation) at the nitrogen atom or the alpha carbon of the amide bond linking two amino acids;
- modifications at the alpha carbon of the amide bond linking two amino acids such as e.g. acylation (preferably acetylation) or alkylation (preferably methylation) at the alpha carbon of the amide bond linking two amino acids.
- chirality changes such as e.g. replacement of one or more naturally occurring amino acids (L enantiomer) with the corresponding D-enantiomers;
- retro-inversions in which one or more naturally-occurring amino acids (L-enantiomer) are replaced with the corresponding D-enantiomers, together with an inversion of the amino acid chain (from the C-terminal end to the N-terminal end);
- azapeptides, in which one or more alpha carbons are replaced with nitrogen atoms; and/or
- betapeptides, in which the amino group of one or more amino acid is bonded to the β carbon rather than the α carbon.

The polypeptide of the invention may also be a recombinant polypeptide, i.e. it has been synthesized using recombinant techniques. In this case, a nucleic acid encoding said polypeptide (further referred to as "a nucleic acid according to the invention") is cloned into an expression vector. The nucleic acid of the invention is preferably placed under the control of expression signals (e.g. a promoter, a terminator and/or an enhancer) allowing its expression. The expression vector is then inserted into a host cell (e.g. a bacterial such as E. coli, a yeast such as Pichia pastoris, a plant, an insect, or a mammalian host cell), and the resulting host cell is cultivated under conditions suitable for the expression of the polypeptide. A recombinant allergen typically only represents one isoform of an allergen. In a preferred embodiment the allergen is a recombinant allergen. In a particular embodiment the allergen is a recombinant low IgE-binding mutant.

The polypeptide of the invention may further include one or more tag(s), which may facilitate its purification.

### Nucleic acids, vectors, host cells and method of producing the polypeptides

An isolated nucleic acid which comprises or consists of a sequence encoding a polypeptide according to the invention, such as for instance a sequence encoding SEQ ID NO: 1, a sequence encoding amino acids 23-386 of SEQ ID NO:28, or a sequence encoding SEQ ID NO: 28, in particular said sequence comprising or consisting of SEQ ID NO: 29, also form part of the invention. A vector comprising a nucleic acid sequence encoding a polypeptide according to the invention operatively associated with expression control elements, and a host cell containing said vector further form part of the invention.

Isolated nucleic acids of the invention, also named polynucleotides, may be DNA or RNA molecules, that encode the polypeptide defined above, while taking into account the degeneracy of the genetic code. They can be obtained by standard techniques well known by the one skilled in the art, such as in vitro DNA amplification or polymerisation, in vitro gene synthesis, oligonucleotides ligation, or by a combination of these techniques.

The nucleic acids of the invention are advantageously in isolated or purified form. By "purified" and "isolated" have the same meaning as defined above.

As will be understood by those of skill in the art, it may be advantageous in some instances to produce polypeptide-encoding nucleotide molecules possessing codons non-naturally occurring in the encoded polypeptide. For example, codons preferred by a particular prokaryotic or eukaryotic host can be selected to increase the rate of recombinant polypeptide expression. It also may be advantageous, for example using site-directed mutagenesis, to alter N-glycosylation sites, enzymatic active sites, B or T-cells epitopes, including IgE binding epitopes.

A nucleic acid according to this invention can also include sequences encoding tags, carriers proteins, signal peptides, or non transcribed or translated sequences increasing expression or stability of the molecule.

The invention also provides nucleic acid sequences that are hybridizable to Amb a X coding sequences or its complementary sequences under standard hybridization conditions, preferably conditions of high stringency.

A nucleic acid molecule is "hybridizable" to another nucleic acid molecule, when a single stranded form of the nucleic acid molecule can anneal to the other nucleic acid molecule under the appropriate conditions of temperature and solution ionic strength (see Sambrook et al., 1989). The conditions of temperature and ionic strength determine the "stringency" of the hybridization. Hybridization requires that the two nucleic acids contain complementary sequences, although depending on the stringency of the hybridization, mismatches between bases are possible. The appropriate stringency for hybridizing nucleic acids depends on the length of the nucleic acids and the degree of complementation, variables well known in the art. For hybrids of greater than 100 nucleotides in length, equations for calculating Tm have been derived (see Sambrook et al., supra, 9.50-9.51). A minimum length for a hybridizable nucleic acid is at least about 10, 11, 12, 13, 14, 15, 16, 17, 18, 19 nucleotides ; preferably at least about 20, 21, 22, 23, 24, 25 nucleotides ; and more preferably the length is at least about 26, 27, 28, 29, 30 nucleotides.

In a specific embodiment, the term "standard hybridization conditions" refers to a Tm of 55°C, and utilizes conditions as set forth above. In a preferred embodiment, the Tm is 60°C or even preferred 65°C. In a specific embodiment, "high stringency" refers to hybridization and/or washing conditions at 68°C in 0.2 X SSC, at 42°C in 50 % formamide, 4 X SSC, or under conditions that afford levels of hybridization equivalent to those observed under either of these two conditions.

In particular, the hybridizable nucleic acids according to the invention may be primers or probes. Thus the invention also concerns primers and probes that are hybridizable to the nucleic acid of the invention under high stringency hybridization conditions.

As used herein, the terms "primer" and "probe" refer to the function of the nucleic acid. A primer is an oligonucleotide used for amplifying a target sequence typically by extension of the oligonucleotide after hybridization to the target sequence or by ligation of multiple oligonucleotides which are adjacent when hybridized to the target sequence. A probe oligonucleotide is used to capture or detect a target sequence to which it hybridizes. However the same oligonucleotide probe may also function as a primer. It will therefore be appreciated that any of the sequences disclosed herein for amplification, detection or quantitation of a nucleic acid may be used either as hybridization probes or as amplification primers for detection or amplification.

The primers and probes of the invention may advantageously be used to amplify a nucleic acid, such as e.g. a nucleic acid encoding the polypeptide of the invention. Said primers and probes may also be used for detecting nucleic acid sequences homologous to the nucleic acid of the invention in a plant species other than *Ambrosia artemisiifolia,* in particular in a related plant species.

Such primers may be for instance primers consisting of a sequence selected from the group consisting of SEQ ID NO: 2 to 26.

The primer or probe of the invention may possibly comprise additional sequences linked to the 5' and/or 3' terminus of the sequence hybridizable to the nucleic acid of the invention, such as a labelling molecule. Said oligonucleotides are nevertheless capable of hybridizing under high stringency conditions with the complementary nucleic sequences of the invention. For instance, these additional sequences may serve as a spacer, linker, or sequence for labelling or binding of an enzyme.

Labelling of the probe is particularly advantageous to facilitate the detection of the amplified nucleic acid, during a "real-time" amplification/detection reaction, i.e. during a PCR process wherein the target sequence is detected and/or quantified while the amplification reaction is occurring. Standard labelling agents (e.g. enzyme, radioactive, or fluorescent moieties) may be used for that purpose.

Such detection may be achieved for instance using the nucleic acid Molecular Beacon technology (Tyagi and Kramer, 1996; Cayouette et al., 1999). According to the Molecular Beacon technology, one of either a fluorophore or quencher moiety is attached to each termini of the probing sequence. In the absence of the target nucleic acid, the arm sequences anneal to each other to thereby form a loop and hairpin stem structure which brings the fluorophore and quencher together. When contacted with target nucleic acid, the complementary probing sequence and target sequence will hybridize. Because the hairpin stem cannot coexist with the rigid double helix that is formed upon hybridization, the resulting conformational change forces the arm sequences apart and causes the fluorophore and quencher to be separated. When the fluorophore and quencher are separated, the fluorescent signal is detectable.

All dyes and quenchers known in the art can be used. According the invention, the dye may be preferably selected from the group consisting of Fam, Tet, Hex, Tamra, Texas Red and Cy5, and the quencher may be preferably selected from the group consisting of Dabcyl, Eclipse Dark Quencher, and Black Hole Quenchers. Such molecules are readily available from Eurogentec, Biosearch Technology, Prolig.

Typically, the nucleic acid of the invention may be included in any suitable vector, such as a plasmid, cosmid, episome, artificial chromosome, phage or a viral vector.

The terms "vector", "cloning vector" and "expression vector" mean the vehicle by which a DNA or RNA sequence (e.g. a foreign gene) can be introduced into a host cell, so as to transform the host and promote expression (e.g. transcription and translation) of the introduced sequence.

The expression vector according to the invention may comprise a functional expression cassette which is also an object of the present invention. An expression cassette comprises a nucleic acid sequence encoding a polypeptide of the invention, which is operably linked to elements necessary to its expression. Said vector advantageously contains a promoter sequence, signals for initiation and termination of translation, as well as appropriate regions for regulation of translation, such as a promoter, enhancer, terminator and the like, to cause or direct expression of said polypeptide upon administration to a subject. Examples of promoters and enhancers used in the expression vector for animal cell include early promoter and enhancer of SV40 (Mizukami T. et al. 1987), LTR promoter and enhancer of Moloney mouse leukemia virus (Kuwana Y et al. 1987), promoter (Mason JO et al. 1985) and enhancer (Gillies SD et al. 1983) of immunoglobulin H chain and the like.

Insertion of said vector into the host cell may be transient or stable. The vector may also contain sequences encoding specific signals which trigger the secretion of the translated protein or its targeting to cellular compartments or organelles (e.g; Golgi apparatus, endosomes, periplasm...). These various control signals are selected according to the host cell and may be inserted into vectors which self-replicate in the host cell, or into vectors which integrate the genome of said host.

Any expression vector for animal cell can be used. Examples of suitable vectors include pAGE107 (Miyaji H et al. 1990), pAGE103 (Mizukami T et al. 1987), pHSG274 (Brady G et al. 1984), pKCR (O'Hare K et al. 1981), pSG1 beta d2-4-(Miyaji H et al. 1990) and the like.

Other examples of plasmids include replicating plasmids comprising an origin of replication, or integrative plasmids, such as for instance pUC, pcDNA, pBR, and the like.

Other examples of viral vector include adenoviral, retroviral, herpes virus and AAV vectors. Such recombinant viruses may be produced by techniques known in the art, such as by transfecting packaging cells or by transient transfection with helper plasmids or viruses. Typical examples of virus packaging cells include PA317 cells, PsiCRIP cells, GPenv+ cells, 293 cells, etc. Detailed protocols for producing such replication-defective recombinant viruses may be found for instance in WO 95/14785, WO 96/22378, US 5,882,877, US 6,013,516, US 4,861,719, US 5,278,056 and WO 94/19478.

A further object of the present invention relates to a cell which has been transfected, infected or transformed by a nucleic acid and/or a vector according to the invention. Consequently, the present invention further concerns a host cell containing a nucleic acid and/or a vector according to the invention, as well as progeny and/or derivatives of such host cells.

The term "transformation" means the introduction of a "foreign" (i.e. heterologous) gene, DNA or RNA sequence to a host cell, so that the host cell will express the introduced gene or sequence to produce a desired substance, typically a protein or enzyme coded by the introduced gene or sequence.

The nucleic acids of the invention may be used to produce a recombinant polypeptide of the invention in a suitable expression system. The term "expression system" means a host cell and compatible vector under suitable conditions, e.g. for the expression of a protein coded for by foreign DNA carried by the vector and introduced to the host cell.

Host cells may be prokaryotic or eukaryotic, including but not limited to bacteria, yeasts, plant cells, animal cells, insect cells, mammalian cells, including cell lines which are commercially available. Preferred examples for expression hosts are *Escherichia coli, Lactobacilli,* probiotic bacteria, *Pichia pastoris, Saccharomyces cerevisiae,* insect cells, plant cells, in particular tobacco plant cells, COS cells and CHO cells.

Common expression systems include *E. coli* host cells and plasmid vectors, insect host cells and Baculovirus vectors, and mammalian host cells and vectors. Specific examples include *E. coli, Kluyveromyces* or *Saccharomyces* yeasts, mammalian cell lines (e.g., Vero cells, CHO cells, 3T3 cells, COS cells, etc.) as well as primary or established mammalian cell cultures (e.g., produced from lymphoblasts, fibroblasts, embryonic cells, epithelial cells, nervous cells, adipocytes, etc.). Examples also include mouse SP2/0-Agl4 cell (ATCC CRL1581), mouse P3X63-Ag8.653 cell (ATCC CRL1580), CHO cell in which a dihydrofolate reductase gene (hereinafter referred to as "DHFR gene") is defective (Urlaub G et al; 1980), rat YB2/3HL.P2.G11.16Ag.20 cell (ATCC CRL1662, hereinafter referred to as "YB2/0 cell"), and the like.

The transfection of the host cell may be performed using any standard technique, such as chemical transformation, electroporation, phosphate calcium precipitation or lipofection.

The present invention also relates to a method for preparing a polypeptide according to the invention, said method comprising:
a) culturing a host cell according to the invention under conditions suitable to obtain expression of a polypeptide according to the invention; and
b) recovering the expressed polypeptide.

The recombinant polypeptide can then be purified, by means of well-known procedures for purification: it may be purified from lysates or cell extracts, inclusion bodies or from the culture supernatant by methods such as HPLC chromatography, immunoaffinity techniques with specific antibodies, and the like.

Alternatively, the polypeptide according to the invention may be expressed in vitro with a cell-free transcription and translation system from a DNA or RNA matrix containing required elements for its expression in a cell lysate or reconstituted system (for example, Rapid Translation System®, Roche Diagnostics or Retic Lysate IVT™, Ambion).

The invention also pertains to an organism, preferably a plant, for instance a tobacco plant, which has incorporated in its genome, advantageously in a stable manner, a nucleic acid molecule of the invention placed under regulation sequence control in a manner to express the polypeptide according to the invention in a plant or a determined part of the plant, such as the fruit, seed, grain, pollen, leaf or tuber. The organism according to the invention may for instance be a field crop plant (wheat, rapeseed, sunflower, peas, soybean, barley, maize, etc.) or a vegetable or flower. Transgenic plants according to the invention can be prepared by transforming a plant cell with the nucleic acid molecule, and then regenerating a plant from the transformed cell.

The invention also pertains to an in vivo method for preparing a polypeptide as defined in any one of claims 1 to 3, said method comprising:
a) cultivating a plant transformed by a nucleic acid of the invention or a vector of the invention under conditions and for a sufficient length of time to enable expression of said polypeptide, and
b) isolating polypeptides produced from the transformed organisms.

The nucleic acid of the invention may be inserted into a nucleic acid construct, called "expression cassette", and is functionally linked to elements which allow the expression thereof and, optionally, the regulation thereof. Among these elements, mention may be made of promoters, activators and terminators of transcription.

Use may preferentially be made of a constitutive promoter, such as the rice actin promoter, followed by the rice actin intron (RAP-RAI) contained in the plasmid pAct1-F4 or the 35S promoter, or a tissue-specific promoter. By way of example, mention may be made of the wheat HMWG promoter or the radish cruciferin gene promoter, PCRU, which both allow expression of the protein of interest in the seeds. Use may advantageously be made of promoter sequences which induce expression under water conditions. Among the terminators which can be used in the constructs of the invention, mention may in particular be made of the 3' end of the *Agrobacterium tumefaciens* nopaline synthase gene. Mention may also be made of the 35S polyA terminator of the cauliflower mosaic virus (CaMV).

The expression of the polypeptide of the invention can also be regulated by using sequences such as peptide addressing signals (chloroplast addressing signals, vacuolar addressing signals, addressing signals for endoplasmic retention, etc.), or such as intron sequences, enhancer sequences or leader sequences.

The expression cassette may be inserted into a nucleotide vector, such as a plasmid, which may also comprise a marker gene, for example a gene making it possible to select between a transformed plant and a plant which does not contain the transfected foreign DNA. As marker gene, mention may be made of a gene which confers resistance to an antibiotic, for example to hygromycin or resistance to a herbicide such as the sulfonamide asulam.

This vector or any sequence encoding the polypeptide of the invention can be used to transform plant cells according to techniques commonly known to those skilled in the art, and then a plant may be regenerated from the transformed cell, said plant expressing the polypeptide of the invention.

The plant cells may be transformed with a vector as defined above, transferred into a cellular host capable of infecting said plant cells by allowing integration into the genome of the latter of the nucleotide sequences of interest initially contained in the genome of said vector. Advantageously, the cellular host used is a bacterial strain, such as *Agrobacterium tumefaciens,* in particular according to the method described in the article by An et al. (1986), or else *Agrobacterium rhizogenes,* in particular according to the method described in the article by Guerche et al. (1987).

For example, the plant cells can be transformed by transferring the T region of the *Agrobacterium tumefaciens* extrachromasomal, circular, tumor-indicating Ti plasmid, using a binary system (Watson et al., 1994). To do this, two vectors are constructed. In one of these vectors, the T region has been removed by deletion, with the exception of the left and right borders, a marker gene being inserted between them so as to allow selection in the plant cells. The other partner of the binary system is a helper Ti plasmid, which is a modified plasmid which no longer has a T region but which still contains the *vir* virulence genes required for transformation of the plant cell.

Use may be made of the method described by Ishida et al. (1996), for the transformation of monocotyledons.

Methods of direct gene transfer into plant cells, such as direct microinjection into plant embryoids (Neuhaus et al., 1987), infiltration under vacuum (Bechtold et al., 1993) or electroporation (Chupeau et al., 1989), or else direct precipitation using PEG (Schocher et al., 1986) or bombardment with particles covered with the plasmid DNA of interest, using a particle gun (M. Fromm et al., 1990) may also be used.

According to another protocol, the transformation is carried out according to the method described by Finer et al. (1992), using a tungsten or gold particle gun.

In the particular case of the expression of recombinant polypeptides in tobacco plant cells, the cells expressing the polypeptide of interest are selected by immunodetection using an antibody according to the invention. The recombinant polypeptides are localized by cell fractionation. Then they are purified from transgenic cell suspensions by immunodetection with an antibody according to the invention. The method according to the invention may also comprise the structural and immunologic analysis of the polypeptides produced.

The invention also provides a transgenic non-human mammal which has incorporated in its genome, advantageously in a stable manner, a nucleic acid molecule of the invention placed under regulation sequence control in a manner to express the polypeptide according to the invention. Preferably, the transgenic non-human mammal of the invention expresses the polypeptide of the invention in its milk. For instance, the transgenic non-human mammal of the invention may comprise a transgene comprising the nucleic acid of the invention operably linked to at least one regulatory sequence which promotes expression of the nucleic acid of the invention in mammary gland cells of the transgenic non-human mammal, and a nucleic acid encoding signal peptide functional in mammary secretory cells of the transgenic non-human mammal of the invention. In an adult form of the non-human mammal or in a female descendant of the transgenic non-human mammal, the transgene is capable of expressing a recombinant polypeptide of the invention in the mammary cells and of producing a form of the polypeptide of the invention which is secreted by the mammary secretory cells into milk of the transgenic non-human mammal. The secreted polypeptide of the invention may further be purified from milk.

Thus, the invention also pertains to an in vivo method for preparing a polypeptide as defined in any one of claims 1 to 3, said method comprising:
a) breeding transgenic non-human mammal transformed by a nucleic acid of the invention or a vector of the invention under conditions and for a sufficient length of time to enable expression of said polypeptide, and
b) isolating polypeptides produced from the transformed organisms.

### Antibodies and uses thereof

The present invention further concerns an isolated antibody which binds specifically to a polypeptide according to the invention.

As used herein the terms "antibody" and "immunoglobulin" have the same meaning and are used in the broadest sense and specifically covers intact monoclonal antibodies, polyclonal antibodies, chimeric, humanized or human antibodies, antibodies, diabodies, multispecific antibodies (e.g. bispecific antibodies) formed from at least two intact antibodies, and also antibody fragments.

In particular, the antibody of the invention may be comprised in an anti-serum.

The invention pertains in particular to an antibody directed against the sequence SEQ ID NO: 30, or against the sequence SEQ ID NO: 31, or against any of the epitopic fragments identified with the Syfpeithi algorithm, in particular a fragment of one of the sequences SEQ ID NO: 32 to 41.

In natural antibodies, two heavy chains are linked to each other by disulfide bonds and each heavy chain is linked to a light chain by a disulfide bond. There are two types of light chain, lambda (λ) and kappa (κ). There are five main heavy chain classes (or isotypes) which determine the functional activity of an antibody molecule: IgM, IgD, IgG, IgA and IgE. Each chain contains distinct sequence domains. The light chain includes two domains, a variable domain (VL) and a constant domain (CL). The heavy chain includes four domains, a variable domain (VH) and three constant domains (CH1, CH2 and CH3, collectively referred to as CH). The variable regions of both light (VL) and heavy (VH) chains determine antigen binding site and thus the recognition and specificity to the antigen. The constant region domains of the light (CL) and heavy (CH) chains confer important biological properties such as antibody chain association, secretion, trans-placental mobility, complement binding, and binding to Fc receptors (FcR). The Fv fragment is the N-terminal part of the Fab fragment of an immunoglobulin and consists of the variable portions of one light chain and one heavy chain.

The term "monoclonal antibody" or "mAb" as used herein refers to an antibody molecule of a single amino acid composition, that is directed against a specific antigen and which may be produced by a single clone of B cells or hybridoma. Monoclonal antibodies may also be recombinant, i.e. obtained by protein engineering. Recombinant antibodies may be produced in a mammalian cell line such as CHO, NSO, PERC6 or any other cell after transfection.

The term "polyclonal antibodies" refers to a combination of immunoglobulins directed against a specific antigen, each immunoglobulin possibly binding to a different epitope on the antigen. Polyclonal antibodies are generally produced by immunisation of a suitable mammal, such as a mouse, rabbit or goat.

The term "chimeric antibody" refers to an engineered antibody which comprises a VH domain and a VL domain of an antibody derived from a non-human animal, in association with a CH domain and a CL domain of another antibody, in particular a human antibody. The non-human animal may be a mouse, a rat, a hamster, a rabbit or the like.

The expression "bispecific antibody" refers to an engineered antibody possessing two different antigen binding sites. In a preferred embodiment of the invention, the at least one CD5 binding molecule and at least one HLA-DR binding molecule of the invention is a bispecific antibody which is able to bind to CD5 and to HLA-DR.

The term "diabodies" refers to small antibody fragments with two antigen-binding sites, which fragments comprise a heavy-chain variable domain (VH) connected to a light-chain variable domain (VL) in the same polypeptide chain (VH-VL). In general, by using a linker that is too short to allow pairing between the two domains on the same chain, the domains are forced to pair with the complementary domains of another chain and create two antigen-binding sites. Preferably the diabody is able to recognize CD5 and HLA-DR.

The expression "humanized antibody" preferably refers to antibodies in which the framework or "complementarity determining regions" (CDR) have been modified to comprise the CDR from a donor immunoglobulin of different specificity as compared to that of the parent immunoglobulin. In a preferred embodiment, a mouse CDR is grafted into the framework region of a human antibody to prepare the "humanized antibody". The antibodies of the invention are preferably "humanized antibodies".

The expression "human antibody" preferably refers to fully human antibodies that have been 1) prepared by immunization in mice with a human immunoglobulin gene repertoire, or 2) prepared by immunization in various strains of immunodeficient mice reconstituted with human immune/hematopoietic cells or 3) to human antibodies isolated from B cells of immunized individuals and EBV transformed or 4) from combination of genes obtained from human VH and VL libraries.

The expression "antibody fragments" comprise a portion of an intact antibody, preferably the antigen binding or variable region of the intact antibody. Examples of antibody fragments include Fv, Fab, F(ab')2, Fab', dsFv, scFv, sc(Fv)2, diabodies and multispecific antibodies formed from antibody fragments.

Another aspect of the present invention concerns an in vitro method for detecting a polypeptide according to the invention in a sample, said method comprising the steps consisting of:
a) incubating a sample with an antibody according to the invention;
b) detecting the presence or absence of immune complexes comprising said antibody;
wherein the presence of immune complexes comprising said antibody is indicative of the presence of a polypeptide according to the invention in said sample.

The "sample" may be an allergen extract, in particular a ragweed pollen allergen extract, or a pharmaceutical composition likely to contain the polypeptide of the invention and which may be intended for diagnosing and/or preventing or treating an allergy. In particular, the pharmaceutical composition may comprise a polypeptide according to the invention which has been recombinantly produced.

The skilled person may use any appropriate qualitative or quantitative method known in the art to detect the presence or absence of immune complexes. In particular, "detecting the presence of immune complexes comprising the antibody according to the invention" may denote detecting the level of, i.e. measuring the amount or concentration of the polypeptide of the invention in a sample. Such immune complexes may readily be detected using for instance a secondary antibody which is an anti-immunoglobulin antibody.

The assay may be carried out by immobilizing the polypeptide on a solid phase, or conversely with the polypeptide in the fluid phase.

The "indirect RAST" or "RAST inhibition" assay may be used for assessing the allergenic activity of an aqueous allergen preparation, for instance for standardizing an allergen extract preparation. In this indirect RAST, the binding of allergen specific IgE to solid phase-allergens is inhibited by addition of free allergen into the test solution. The degree of inhibition can then be used to measure the biological activity exerted by the free allergen (Puttonen et al., Clin Allergy 1981;11 (2):139-45). The principle of RAST assay is described in further details in the "Diagnostic applications" section.

In the "AIRAST" (Aluminium hydroxide RAST) test, the solid-phase sorbent is replaced by a gel of aluminium hydroxide, thus avoiding the covalently binding used in the conventional RAST. Such test may be advantageously used when covalent binding of the allergen to the solid-phase sorbent may mask some of its antigenic determinants (Poulsen et al., Allergy 1985;40(6):405-16).

In particular, "detecting the presence or absence of immune complexes comprising said antibody" may comprise the steps of detecting the level of immune complexes comprising the antibody according to the invention and comparing said level to a reference level.

Furthermore, the present invention also provides a method for quantifying the immunological activity of an allergen extract or an anti-allergy vaccine preparation.

For instance, "RAST inhibition" may be used for the standardization of allergen preparations. Test systems may be established in conjunction with investigation of the immunological activity of an allergen reference preparation.

Said "allergen reference preparation" may for instance be an allergen extract, an anti-allergy vaccine preparation, or a preparation involving quantitative skin prick test in 20 allergic subjects with a proven history of allergic disease. Sera from these patients may then be characterized to establish that they cover the full spectrum of allergen sensitivities and may be used to create a serum pool that, together with the allergen reference preparation, form the basis of the test system.

The immunological activity of an allergen extract or an anti-allergy vaccine preparation to be quantified may then be evaluated and standardized in relation to the reference preparation using the test system. The relative potency may for instance be defined in terms of the 50% inhibition value. Parallel regression lines may provide one indication that the preparations contain comparable spectra of allergenic determinants.

The allergen extract or the anti-allergy vaccine preparation to be analysed may in particular comprise physically modified allergen following adsorption to carriers, such as aluminium hydroxide and calcium phosphate, or formulated allergens, for example after encapsulation in micro-particles. The immunological activity of said extract or preparation may be evaluated directly by RAST inhibition, which gives an indication of the overexpression of IgE-binding epitopes. The preparation may in particular be compared with the unmodified allergen extract or preparation, standardized relative to the native allergen reference preparation, and re-evaluated at intervals to establish stability. The relative potency of the modified preparation may appear to be less than that of the starting material, but this can be explained in terms of steric hindrance of epitopes by binding to the solid phase.

Besides, the activity of extracts or preparations comprising chemically modified allergens may also be assessed by RAST inhibition for the purposes of quality control. For instance, formaldehyde treatment of allergen extracts or preparations may result in substantial reductions in their IgE-binding capacity, by e.g. 50 to 1000-fold depending on the allergen extract. RAST inhibition may thus be used to assess the stability of the chemical modification, as an increase in IgE-binding activity with time may be indicative of a reversal of the chemical modification.

Furthermore, the present invention also provides an in vitro method for quantifying a polypeptide according to the invention in a sample, said method comprising the steps consisting of:
a) providing a known amount of the polypeptide as defined in claim 1, optionally labelled, as a calibration standard,
b) degrading the sample containing the polypeptide to be quantified to obtain a mixture of polypeptides, optionally labelled,
   wherein at least the polypeptides in the degraded sample or in the calibration standard are labelled, and if both polypeptides are labelled, the labelling agent used for the polypeptides in the calibration standard is different from the labelling agent used for the polypeptides in the degraded sample,
c) quantifying the absolute amount of the polypeptide according to the invention in the sample by correlating the amount of the polypeptide in the calibration standard with the amount of the corresponding polypeptide in the degraded sample by mass analysis.

The quantification step may for instance be performed using mass spectrometry, labelling may for example be performed using ITRAQ™ chemistry, and the degradation step may for instance be performed by using a proteolytic enzyme, such as trypsin, papain, pepsin, ArgC, LysC, V8 protease, AspN, pronase, chymotrypsin and carboxypeptidase C, or a combination thereof, as described in WO 2007/031080.

The calibration standard polypeptide may be a polypeptide with an amino acid sequence identical to, either a variable or a constant sequence in a group of isoallergens or homologous allergens, depending on whether it is to be used for quantification of an allergen consisting of more than one isoallergens or homologous allergens or a specific allergen or isoallergen. In the case of quantification of the absolute amount of allergen in a sample, the calibration standard polypeptide may be an amino acid sequence region which is constant, i.e. identical in the group of isoallergens of the allergen or in the homologous allergens to be quantified. In the case of quantification of a specific allergen or isoallergen in a sample, the calibration standard polypeptide may be an amino acid sequence region which is variable, i.e. unique for the isoallergen or the allergen which is to be quantified.

The allergen calibration peptide is preferably prepared by peptide synthesis. The number of amino acids in the allergen calibration standard peptide is preferably in the range of 2-20 amino acids, more preferred in the range of 4-15 and most preferred in the range of 6-15. The number is dependent on the optimal enzymatic cleavage site found to match to the amino acid sequence within the sample i.e the constant or the variable region sequence when the sample is cleaved by an enzyme. Furthermore, the allergen calibration standard to be used depends on the label and the quantification method to be used in order to give a detectable signal and fragmentation when analysed in a MS instrument.

### Therapeutic applications

The present invention also relates to a pharmaceutical composition comprising a polypeptide or an antibody according to the invention and a pharmaceutically acceptable carrier.

The present invention also concerns a polypeptide or an antibody according to the invention for use as a medicament.

The present invention further relates to a polypeptide or an antibody according to the invention for use for preventing or treating an allergic reaction to ragweed pollen.

The antibody to be used according to the invention is preferably an IgG antibody.

Polypeptide, preferably SEQ ID NO:1 or isoallergen or isoform thereof, variant with reduced allergenicity and/or enzymatic activity, or derivative

Preferably, the pharmaceutical compositions, the polypeptides or the antibodies of the invention are used to treat immediate allergies, or mastocytosis.

The term "immediate allergy" or "type I hypersensitivity" as used herein means an antibody response in response to an allergen that is different from a normal humoral response to the fact that plasma cells secrete IgE.

Among the clinical manifestations caused by immediate allergy treatable by the pharmaceutical compositions, polypeptides, or antibodies of the invention include, for example, systemic anaphylaxis, localized anaphylaxis (atopy), allergic rhinitis, asthma, atopic dermatitis, conjunctivitis, eczema, mastocytosis induced anaphylactic shock.

According to the invention, the allergy to be treated is caused by exposure of an individual to the allergen Amb a X, or to a homologous protein or polypeptide as defined above, in particular an isoallergen thereof, an isoform thereof, or by exposure to a pollen containing one of them.

The "pharmaceutical composition" may be an "immune composition" or a "vaccinal composition". As used herein, the term "immune composition" denotes a composition which is liable to induce an immune response when administered in an individual. As intended herein, the term "vaccinal" relates to the capacity of a substance to prevent or to treat a pathological reaction of the immune system.

In the context of the invention, the terms "to treat", "treating" or "treatment", means reversing, alleviating, or inhibiting the course of a pathological reaction of the immune system or one or more symptoms thereof. In the context of the invention, the terms "to prevent" or "preventing", means the onset of a pathological reaction of the immune system or one or more symptoms thereof.

The term "allergic reaction" refers to any kind of abnormally hypersensitive response of the immune system to certain substances, such as pollens, foods, or microorganisms. Such substances that cause a reaction are called allergens. Common indications of allergy may include sneezing, itching, and skin rashes. Allergic reactions are often associated with excessive production of immunoglobulins E (IgEs) and activation of mast cells and basophils.

As used herein, the term "individual" preferably denotes a human, but may more generally a mammal, such as a rodent, a feline, a canine, and a primate.

The suitable immune or vaccinal compositions may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

The pharmaceutical composition may further comprise at least one other active agent, in particular at least another allergen such as an allergen from pollen, an allergen from food, an allergen from house dust, an allergen from mites, an allergen from molds, an allergen from venom, or an allergen from animal dander, animal hair, animal fur or animal saliva, as described in further details in the section relating to diagnostic applications here below.

The pharmaceutical composition may for example comprise at least two different types of allergens either originating from the same allergic source or originating from different allergenic sources.

"Pharmaceutically" or "pharmaceutically acceptable" refers to molecular entities and compositions that do not produce adverse, allergic or other untoward reactions when administered to an animal, or a human, as appropriate.

As used herein, the term "pharmaceutically acceptable excipient" includes solvents, dispersion media, coatings, antibacterial and antifungal agents, isotonic and absorption delaying agents, mucoadhesive excipients, and the like, that do not produce an adverse or other untoward reaction when administered to an animal, or a human, as appropriate. Excipients may further include, but are not limited to disintegrants, binders, lubricants, flavoring, colorants, preservatives. Suitable disintegrants include dry starch, calcium carbonate, polyoxyethylene sorbitan fatty acid esters, sodium lauryl sulfate, stearic monoglyceride, lactose, as well as cross-linked polyvinylpyrrolidones, such as crospovidone (e.g., Polyplasdone™. XL, which may be obtained from GAF), cross-linked carboxylic methylcelluloses, such as croscarmelose (e.g., Ac-di-sol™, which may be obtained from FMC), alginic acid, and sodium carboxymethyl starches (e.g., Explotab™, which may be obtained from Edward Medell Co., Inc.), methylcellulose, agar bentonite and alginic acid. Binders, if used, are those that enhance adhesion. Examples of such binders include, but are not limited to, starch, gelatin and sugars such as sucrose, dextrose, molasses, and lactose. Preferred lubricants are stearates and stearic acid. Lactose, mannitol, and croscarmelose are preferred excipients.

Where mucosal administration is contemplated, the pharmaceutically acceptable excipient may advantageously be a "mucoadhesive carrier". As intended herein, a "mucoadhesive carrier" enables close and prolonged contact with a mucosa, in particular a mucosa of the oral cavity, and more particularly the sublingual mucosa, thereby enhancing-antigen specific tolerance induction. Preferred mucoadhesive carriers as defined herein notably comprise chitosan, polymers of maltodextrin or carboxymethylcellulose.

In the frame of methods for preventing or treating allergic reactions, the pharmaceutical compositions or the medicaments, according to the invention, can include any conventional adjuvant. As intended herein an "adjuvant" enhances antigen-specific tolerance induction. Adjuvants have been used for many years to improve the host immune responses to, for example, vaccines. The adjuvant as defined herein may include any conventional or exploratory, synthetic or biological adjuvant for vaccination, including heat-labile enterotoxin (LT), cholera-toxin (CT), cholera toxin B subunit (CTB), polymerised liposomes, mutant toxins, probiotic bacteria, saponins complexed to membrane protein antigens (immune stimulating complexes), pluronic polymers with mineral oil, killed mycobacteria in mineral oil, Freund's complete adjuvant, bacterial products, such as muramyl dipeptide (MDP) and lipopolysaccharide (LPS), as well as lipid A, or biological or synthetic ligands of Toll like receptors (eg TLR2, 4, 5, 7 or 9).

For oromucosal administration, the adjuvants may preferably be a Bifidobacterium, a lactic acid bacterium (either in the form of a cell suspension, freeze-dried cells, a lysate, purified sub-components, or purified molecules), or a combination of a corticosteroid with vitamin D3 or any metabolite or analog of the latter.

Advantageously, where mucosal administration is contemplated, the adjuvant may be a synthetic particulate vector that comprises a non-liquid hydrophilic core which comprises a cross-linked polysaccharide. Accordingly, the polypeptide according to the invention may be formulated in a mucoadhesive formulation based on a synthetic particulate vector that comprises (i) a particle comprising a non-liquid hydrophilic core which comprises a cross-linked polysaccharide; and (ii) a polypeptide according to the invention. Such a formulation was found to be particularly efficient in inducing immune tolerance. The particles which can be used are described in the international patent application WO 2008/023233.

Briefly, the cross-linked polysaccharide may be derived from any saccharide monomers, preferably glucose. The polysaccharides preferably have a molecular weight between 2,000 to 100,000 daltons, and most preferably 3,000 to 10,000 daltons. Preferred polysaccharides are starch (glucose alpha 1-4 polymers) and dextran (glucose alpha 1-6 polymers derived from bacteria), or hydrolysates thereof such as dextrins or maltodextrins.

Ionic groups, i.e. anionic (e.g. sulfate or carboxylate) or cationic groups (e.g. quaternary ammonium ions, and primary, secondary, or tertiary amines) are optionally grafted to the core of cross-linked polysaccharide (preferably 0 to 3 milliequivalents, more preferably 0 to 2 milliequivalents, of ionic charge per gram).

Optionally, the cross-linked polysaccharide core is at least partially coated with a layer of amphiphilic compounds and/or a layer of lipidic compounds.

The diameter of the particle may be comprised between 10 nm and 5 µm and preferably between 20 and 200 nm.

For parenteral administration in an aqueous solution, for example, the solution should be suitably buffered if necessary and the liquid diluent first rendered isotonic with sufficient saline or glucose. These particular aqueous solutions are especially suitable for intramuscular and subcutaneous administration. In this connection, sterile aqueous media which can be employed will be known to those of skill in the art in light of the present disclosure.

Preferably, the pharmaceutical composition, or the medicament is to be administered by the mucosal route, more preferably by the oromucosal route, and most preferably by the sublingual route. As such the pharmaceutical composition and the medicament are preferably formulated in a way adapted for such administration routes.

Mucosal administration denotes any administration method, wherein the formulation in part or in full comes into contact with a mucosa. Mucosa refers to the epithelial tissue that lines the internal cavities of the body. The mucosal surface may be selected from the group consisting of a nasal, buccal, oral, vaginal, ocular, auditory, pulmonary tract, urethral, digestive tract, and rectal surface.

Oromucosal administration comprises any administration method, wherein the formulation in part or in full comes into contact with the mucosa of the oral cavity and/or the pharynx of the patient. It includes in particular sublingual, perlingual (i.e. through the tongue mucosa) and oral administrations.

Where the medicament is administered sublingually to the patient (i.e. under the tongue), the sublingual mucosa, located on the underside of the tongue, facilitates capture of the antigen and adjuvant by Langerhans-like cells that migrate to draining lymph nodes to prime T lymphocytes. A route of administration of particular interest is to keep the composition under the tongue a few minutes, e.g. about 2 minutes, before swallowing or spitting it out.

The medicaments according to the invention can be administered in various forms, such as dispersed forms, e.g. in suspensions or gels, or as dry forms, e.g. in powders, tablets, capsules, delayed release capsules, lyoc, or forms suitable to be administered in a metered-dosing device. The use of liposomes and/or microparticles and/or nanoparticles is also possible. The use and formation of liposomes and/or microparticles and/or nanoparticles are known to those skilled in the art.

In the frame of methods for preventing or treating allergic reactions, the pharmaceutical compositions or the medicaments, according to the invention, the administration regimen may be maintained for instance for a period of less than 6 weeks to more than 3 years.

Some variation in dosage will necessarily occur depending on the condition of the subject being treated. Dosages to be administered depend on individual needs, on the desired effect and the chosen route of administration. It is understood that the dosage administered will be dependent upon the age, sex, health, and weight of the recipient, concurrent treatment, if any, frequency of treatment, and the nature of the effect desired. The total dose required for each treatment may be administered by multiple doses or in a single dose. The person responsible for administration will, in any event, determine the appropriate dose for the individual subject. For instance, in the frame of methods for preventing or treating allergic reactions, the dose range for the polypeptides of the invention contained in the pharmaceutical compositions or the medicaments according to the invention may be between 1 to 200 µg/day, preferably between 1 to 100 µg/day, more preferably between 1 to 50 µg/day by oral route.

The antibody of the invention is preferably present in an amount of about 1 to 1000 milligrams, preferably of about 50 to 800 milligrams, more preferably of about 75 to 600 milligrams per dose, in a pharmaceutcial composition for subcutaneous administration every 2 or 4 weeks. Multiple doses can also be administered.

### Diagnostic applications

The polypeptide according to the invention may further be used to detect antibodies directed against a ragweed pollen allergen, in particular IgE antibodies, in a sample from an individual. Accordingly, the present invention also relates to a polypeptide according to the invention for use for detecting an allergy or sensitivity to ragweed pollen.

The present invention further provides an in vitro method of diagnosing an allergy or sensitivity to ragweed pollen in an individual, said method comprising the steps consisting of:
a) incubating a polypeptide according to the invention with a biological sample of an individual;
b) detecting the presence or absence of immune complexes between said polypeptide and IgEs from said biological sample of the individual;
wherein the presence of immune complexes between said polypeptide and IgEs from said biological sample of the individual indicates that the individual is sensitized or allergic to ragweed pollen.

The individual may be a human or a non-human animal, in particular a non-human mammal, such as a rodent, a feline, a canine, and a primate.

The biological sample may be in particular a biological fluid, such as blood, plasma or serum.

The skilled person may use any appropriate qualitative or quantitative method known in the art, to detect the antibodies. The assay may be carried out by immobilising the polypeptide on a solid phase, or conversely with the polypeptide is the fluid phase. Typical methods which may be used include ELISA, Western blotting, RAST, RAST inhibition or AIRAST.

The "RAST test" (RadioAllergoSorbent Test) is a test used for diagnosing an allergy. More precisely, the RAST test is a radioimmunoassay test to detect specific IgE antibodies directed to suspected or known allergens. Briefly, the allergen is bound to an insoluble material and the patient's serum is added. If the serum contains antibodies to the allergen, those antibodies will bind to the allergen. Radiolabeled anti-human IgE antibodies are added and bind to the IgE antibodies already bound to the insoluble material. The unbound anti-human IgE antibodies are washed away. The amount of radioactivity is proportional to the serum IgE directed to the allergen. The RAST test is then scored on a scale from 0 to 6.

Where the concentration of the antibodies is determined, quantitation of the antibody response may be repeated in time, for instance in order to monitor efficacy of a desensitization treatment administered to the individual.

The polypeptide according to the invention may further be used for cellular tests such as a T-cell proliferation test, mediator release test etc. The polypeptide may be exposed to various types of cells in order to elicit measurable responses. Such responses may comprise the release of histamine or other mediators (e.g., leukotriens, serotonine, ECP) in the case of allergic effector cells (e.g., basophils mast cells, eosinophils). In another type of assay the proliferation or death (e.g., apoptosis) of cells may be measured e.g., by the uptake of ³H Thymidine or any other suitable assay. Such cells may be T cells. Furthermore, polypeptides may be used to induce the release of cytokines or other immunologically relevant substances (e.g., from T cells) that can be measured. Such cellular tests can be performed for instance on PBMC collected from an individual.

Since polypeptides can contain epitopes of unrelated allergens they may be used for diagnostic screening tests (in vitro, in vivo as outlined above) in order to detect sensitization or unresponsiveness of an individual against one of the components of the polypeptide. This may allow providing the physician with a diagnostic test which is suited to screen for sensitized patients in a fast way.

Thus the polypeptide according to the invention may also be used for diagnostic purposes, for instance for in vivo provocation testing. Such tests may comprise skin testing (e.g., skin prick or intradermal testing), nasal provocation testing, all forms of food challenge testing or bronchial provocation testing.

A "prick test", also known as "skin test", "puncture test" or "scratch test", denotes a method for medical diagnosis of allergies that attempts to provoke a small, controlled, allergic response. Briefly, small amounts of purified allergens and/or their extracts are introduced to sites on the skin marked with pen or dye. A "lancet" denotes a small plastic or metal device which may be used to puncture or prick the skin surface. The allergens may also be injected "intradermally" into the patient's skin, with a needle and syringe. Common areas for testing include the inside forearm and the back. If the patient is allergic to the substance, then a visible inflammatory reaction may occur within 30 minutes. This response can range from slight reddening of the skin to a full-blown hive (called "wheal and flare") in more sensitive patients similar to a mosquito bite. Allergists may then measure and record the diameter of the wheal and flare reaction. Interpretation of the results of the skin prick test may subsequently be done by allergists on a scale of severity.

Accordingly, the present invention also provides a method of diagnosing an allergy or sensitivity to ragweed pollen in an individual. In particular, said method may comprise the steps of (a) injecting by intradermal or subcutaneous route the polypeptide according to the invention; and (b) detecting IgE reactivity, in particular by measuring the diameter of the wheal and flare reaction at the site of injection, wherein an IgE reactivity is indicative of an individual sensitised or allergic to a ragweed pollen allergen.

The invention further provides a method of detecting an IgE reactivity in an individual which comprises the steps of (a) injecting by intradermal or subcutaneous route the polypeptide according to the invention; and (b) detecting a wheal and flare reaction at the site of injection, wherein a wheal and flare reaction is indicative of an IgE reactivity of the individual to a ragweed pollen allergen.

According, the diameter of the wheal and flare reaction at the site of injection may be measured to quantify the IgE reactivity.

In order to practice the above in vivo method of diagnosing an allergy or sensitivity, or of detecting an IgE reactivity, the polypeptide of the invention may be advantageously formulated into a pharmaceutical composition, as described previously in the section relating to therapeutic applications.

The invention also relates to the use of a polypeptide according to the invention, for the manufacture of a diagnostic test. Said diagnostic test further makes part of the invention and is intended to be used for screening of patients sensitized to ragweed pollen allergens.

The invention further concerns a kit for the diagnosis of an allergy, in particular to ragweed pollen, comprising a polypeptide according to the invention and a lancet. Said kit may optionally comprise instructions for use. Alternatively, the kit according to the invention comprises a polypeptide as defined herein and instructions for use. The kit according to the invention may in particular be used to perform a prick test.

In particular, the kit may further comprise one or more environmental allergen, as described below. Allergens are well-known to the skilled in the art. Common environmental allergens which induce allergic diseases are found in pollen (e.g. tree, herb, weed and grass pollen allergens), food, house dust, mite (especially mite feces), animal danders, hair and/or saliva (from e.g. dog, cat, horse, rat, mouse etc.), molds, fungal spores and venoms (for example insect or batracian venom).

Therefore, the other allergen optionally present in the kit according to the invention preferably is an allergen from pollen, an allergen from food, an allergen from house dust, an allergen from mites, an allergen from molds, an allergen from venom, or an allergen from animal dander, animal hair, animal fur or animal saliva. Important pollen allergens from trees, grasses and herbs are such originating from the taxonomic orders of Fagales, Oleales, Pinales and platanaceae including i.a. birch (Betula), alder (Alnus), hazel (Corylus), hornbeam (Carpinus) and olive (Olea), cedar (Cryptomeria and Juniperus), Plane tree (Platanus), the order of Poales including i.a. grasses of the genera Lolium, Phleum, Poa, Cynodon, Dactylis, Holcus, Phalaris, Secale, and Sorghum, the orders of Asterales and Urticales including i.a. herbs of the genera Ambrosia, Artemisia, and Parietaria. Other important inhalation allergens are those from house dust mites of the genus Dermatophagoides and Euroglyphus, storage mite e.g Lepidoglyphys, Glycyphagus and Tyrophagus, those from cockroaches, midges and fleas e.g. Blatella, Periplaneta, Chironomus and Ctenocepphalides, and those from mammals such as cat, dog and horse, venom allergens including such originating from stinging or biting insects such as those from the taxonomic order of Hymenoptera including bees (superfamily Apidae), wasps (superfamily Vespidea), and ants (superfamily Formicoidae). Important inhalation allergens from fungi are i.a. such originating from the genera Alternaria and Cladosporium.

The kit according to the invention may comprise one or more allergens, e.g. up to 2, 3, 4, 5, 6, 7, 8, 9, 10, 15, 20, 25, 50, 75 or 100 different allergens. The kit may for example comprise at least two different types of allergens either originating from the same allergic source or originating from different allergenic sources e.g. grass group 1 and grass group 5 allergens, or mite group 1 and group 2 allergens, from different mite and grass species respectively.

The invention will be further illustrated in view of the following examples.

### BRIEF DESCRIPTION OF THE SEQUENCES

SEQ ID NO: 1 shows the consensus polypeptidic sequence of mature Amb a X.
SEQ ID NO: 2-26 show the sequences of primers (see Table 3).
SEQ ID NO: 27 shows a Kozak-like sequence.
SEQ ID NO: 28 shows the consensus polypeptidic pre-pro-sequence of Amb a X.
SEQ ID NO: 29 shows the consensus nucleic sequence encoding the polypeptidic pre-pro-sequence of Amb a X.
SEQ ID NO: 30-31 show the sequences of peptides used for immunization and production of antibodies.
SEQ ID NO: 32-41 show the sequences of predictive epitopic fragments.

### DESCRIPTION OF THE FIGURES

Figure 1: Principles of the allergen nomenclature

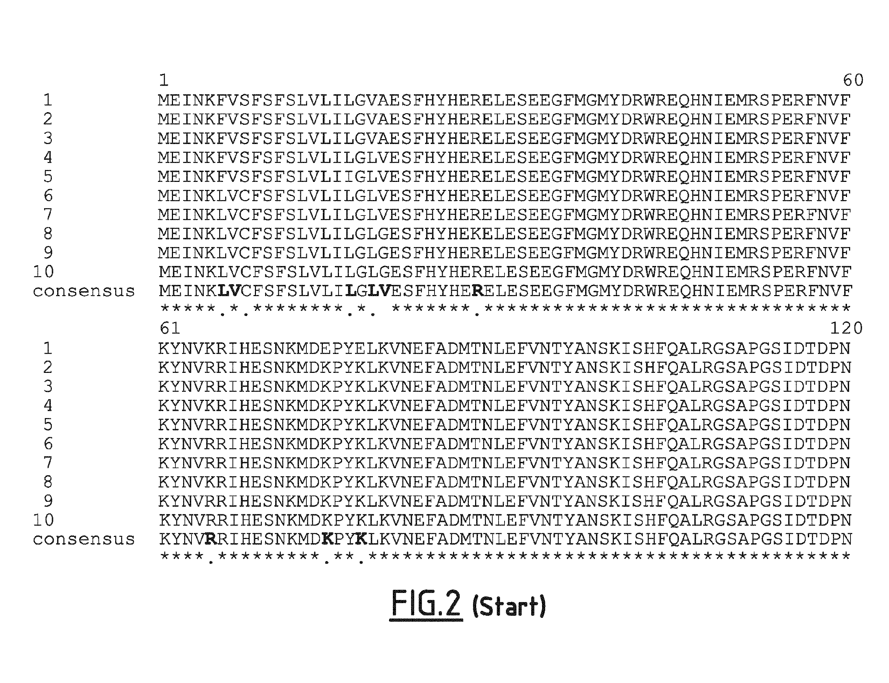

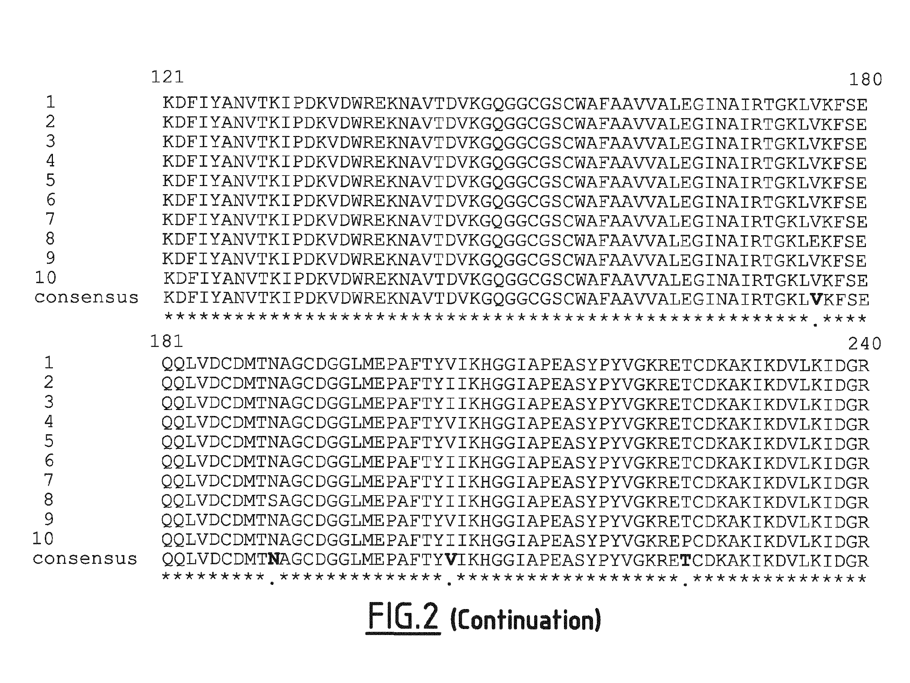

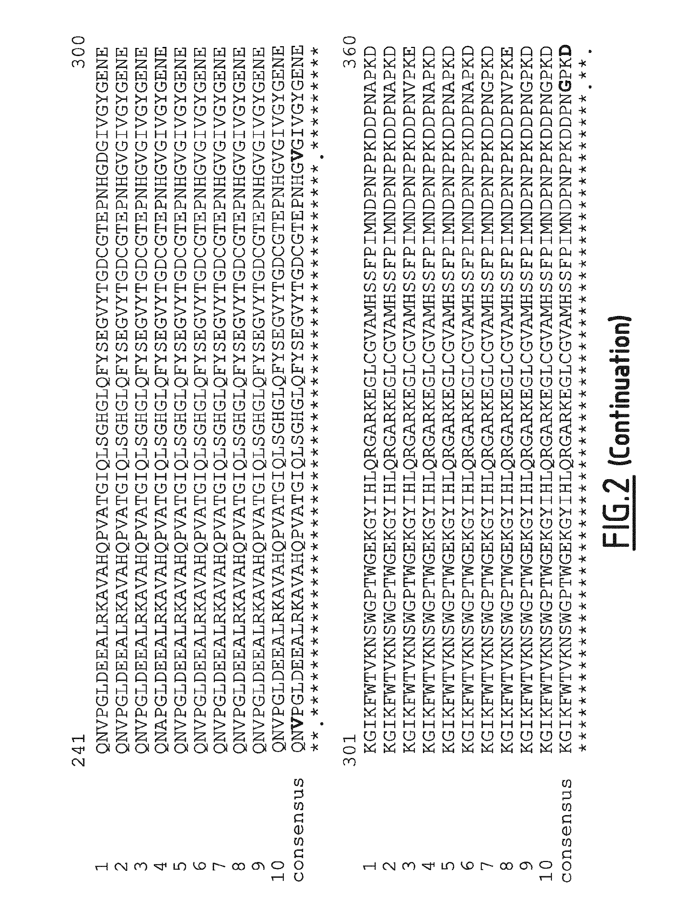

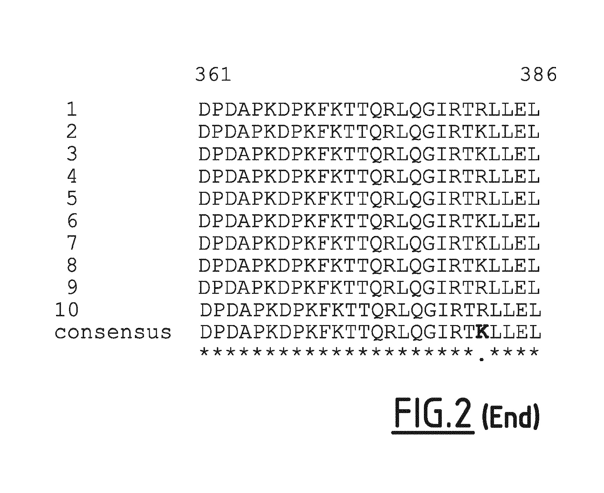

Figure 2: Sequence alignment of 10 clones of the complete polypeptidic pre-pro-sequence of Amb a X with the consensus sequence.

### EXAMPLES

### Example 1: Identification of a new ragweed pollen allergen, named Amb a X

### Example 1.1: Context and objectives

The purpose of this study was to establish the allergenic sensitization profile of 28 well-characterized ragweed-pollen allergic patients selected based upon biologic and clinical parameters. To this aim, the identification of Amb a 1 isoforms of ragweed pollen raw material was first performed by 2D electrophoresis and mass spectrometry. Secondly, IgE reactivity from patients' sera with allergens, most particularly Amb a 1 isoforms, was determined by Western blot analyses.

### Example 1.2: Material and methods

### Example 1.2.1: Material

Sera of 28 ragweed-pollen allergic patients were collected prior to any specific immunotherapy treatment. Sera were stored at -20 °C before use.

### Example 1.2.2: Pollen extraction

Ragweed pollen extract was used and ragweed pollen raw material was purchased from GREER Laboratories (Lenoir, NC, USA).

For preparing ragweed pollen extract, 1 gram of pollen was extracted with 20 mL of ammonium bicarbonate solution at 4 g/L prepared in pure water. Extraction was performed at 4°C overnight with rotative agitation. Centrifugation was performed at 4000 rpm for 15 minutes at 4°C. Supernatant, that represents the pollen extract, was collected, aliquoted and stored at -20 °C before protein quantification assay and use.

### Example 1.2.3: Bidimensional electrophoresis

Total protein extracts were loaded onto Immobiline Dry Strips, pH 3-10 NL 13 cm (GE Healthcare@) over night with 2% of IPG Buffer (GE Healthcare@) and Destreak reagent (GE Healthcare@) at 12 µL/mL of sample volume. In the case of Coomassie Blue staining following the 2D separation, 150 µg of total protein were loaded onto the strip. In the case of transfer and immune blot following the 2D separation, 30 µg of total protein were loaded onto the strip. The first dimension separation was performed with IPGphor3 apparatus (GE Healthcare@) using 4 successive phases (phase 1: 500 V, gradient 1 hour ; phase 2: 1000 V, gradient 1 hour ; phase 3: 8000 V, gradient 2 hour 30 ; phase 4: 8000 V, step-n-hold 3 hours). This first dimension separation was stopped after at least 25000 Vh cumulated voltage. For the second dimension, the strip was equilibrated with lodoacetamide and DTT and loaded onto an ExcelGel 2D Homogenous 12.5% horizontal SDS PAGE (GE Healthcare@) for 1 h 45 (phase 1: 120 V, 35 min ; phase 2: 600 V, 1h10). Separated pollen extract proteins were either treated with Coomassie Staining or transferred onto nitrocellulose membrane.

### Example 1.2.4: Coomassie staining and picking

After 2D gel electrophoresis, gel was incubated in fixation solution (40% ethanol, 10% acetic acid) for 30 minutes. Proteins were then stained with Coomassie (Coomassie brillant blue dye R 250 and coloration solution: PhastGel Blue R: 1 tablet in 400 mL of decoloration solution filtrated and warmed at 60°C) for 10 minutes. A decoloration step was performed (gel incubated in decoloration solution: 25% ethanol, 8% acetic acid) until spots were clearly visible against the clear background. Spots were manually picked from the gel in a protected environment to avoid keratin contamination (gloves, mask, mobcap). Spots were conserved in 30% ethanol at 4°C prior to Mass spectrometry analyses.

### Example 1.3: Results and interpretation

A 2D electrophoresis separation of the ragweed pollen extract was performed, followed by Coomassie staining. Twenty spots were picked from the 2D gel and submitted to a mass spectrometry identification analyses in a set of 3 independent mass spectrometry (MALDI TOF and LC-MS/MS) analyses.

Analyses of 9 out of 20 picked spots showed that the 5 Amb a 1 isoforms were clearly identified within this extract. The remaining eleven spots were also analysed, allowing defining 6 other major proteins with "correct peptide map profile", including an unknown Amb a X allergen. Unfortunately, the complete ragweed proteome has not been achieved and those additional proteins could not be matched with information available in data bases.

Allergenic profiles obtained with the 28 sera were analysed. 54 % of patients' sera were found to react with the unkown allergen named Amb a X in 2D western blot.

### Example 2: RT-PCR cloning of the new ragweed pollen allergen Amb a X

### Example 2.1: Objectives

The aim was to clone, by a RACE approach, the ragweed allergen Amb a X using DNA sequences defined based on peptide sequences determined by 2D electrophoresis followed by mass spectrometry and Edman N-terminal sequencing.

### Example 2.2: Material and methods

### Example 2.2.1: Material

Total RNA were extracted from defatted ragweed pollen. Pollen was first resuspended in water and ground pollen in liquid nitrogen using a small mortar and pestle before RNA extraction.

### Example 2.2.2: Rapid Amplification of cDNA Ends (RACE)

Primers used for RACE experiments are described in table 3.

**Table 3: primers used in RACE experiments**

| **Name** | **Sequence** | **SEQ ID NO** | **Orientation** |
|---|---|---|---|
| **STA201** | AACGCCGTTACCGATGTTAAA | SEQ ID NO: 2 | forward |
| **STA203** | CCAGAAGCCAGCTACCCATAC | SEQ ID NO: 3 | forward |
| **STA208** | ACCCCAGGTTGGACCCCAAGAGTT | SEQ ID NO: 4 | reverse |
| **STA352** | TCTTACCCATACGTTGGTAA | SEQ ID NO: 5 | forward |
| **STA357** | CATGGTGGTATCGCCCCAGAAGCCAGCTACCCATA | SEQ ID NO: 6 | forward |
| **STA363** | CATGGTGGTTTGGCCCCAGAAGCCTCTTACCCATA | SEQ ID NO: 7 | forward |
| **STA368** | CATGGTGGTTTGGCCCCAGAAGCCAGCTACCCATACGTTGGTAA | SEQ ID NO: 8 | forward |
| **STA375** | CATGGTGGTCTCGCCCCAGAAGCCTCTTACCCATA | SEQ ID NO: 9 | forward |
| **STA386** | CAAAACGTTCCAGGTATCGATGAAGAAGCCATCAGAAA | SEQ ID NO: 10 | forward |
| **STA390** | CAAAACGTTCCAGGTATCGATGAAGAAGCCCTCAGAAA | SEQ ID NO: 11 | forward |
| **STA392** | CAAAACGTTCCAGGTTTGGATGAAGAAGCCATCAGAAA | SEQ ID NO: 12 | forward |
| **STA394** | CAAAACGTTCCAGGTCTCGATGAAGAAGCCATCAGAAA | SEQ ID NO: 13 | forward |
| **STA400** | CAAAACGTTCCAGGTCTCGATGAAGAAGCCTTGAGAAA | SEQ ID NO: 14 | forward |
| **STA476** | GATACCGACCCTAATAAAGATTTCATATATGCA | SEQ ID NO: 15 | forward |
| **STA490** | GGCAAAAGAGAAACCTGCGACAAAGCAAAGATT | SEQ ID NO: 16 | forward |
| **STA496** | GGACTTGACGAAGAAGCACTAAGGAAGGCA | SEQ ID NO: 17 | forward |
| **STA497** | TGCCTTCCTTAGTGCTTCTTCGTCAAGTCC | SEQ ID NO: 18 | reverse |
| **STA505** | TCCCACAATTCCAACACCATGATTCGGCTC | SEQ ID NO: 19 | reverse |
| **STA510** | GTCACTGATGTCAAGGGTCAAGGCGGATGTGGA | SEQ ID NO: 20 | forward |
| **STA516** | GTAAAATTTTCCGAACAACAA | SEQ ID NO: 21 | forward |
| **STA517** | TTGTTGTTCGGAAAATTTTAC | SEQ ID NO: 22 | reverse |
| **STA536** | TCAGCACCTGGCTCGATTGATACCGACCCTAATAAAGATTTC | SEQ ID NO: 23 | forward |
| **STA537** | GAAATCTTTATTAGGGTCGGTATCAATCGAGCCAGGTGCTGA | SEQ ID NO: 24 | reverse |
| **STA538** | TCAGCACCTGGCTCGATCGATACCGACCCTAATAAAGATTTC | SEQ ID NO: 25 | forward |
| **STA539** | GAAATCTTTATTAGGGTCGGTATCGATCGAGCCAGGTGCTGA | SEQ ID NO: 26 | reverse |

Total RNA was used as template for 5' and 3' RACE experiments. Reverse transcription and subsequent PCR amplifications were performed starting from 1 µg total RNA. cDNA were used as template for a first round of PCR amplification using universal primers and Amb a X peptidic sequence-derived primers as described in Table 4. Amplification cycles were performed with 50-65°C hybridisation steps. First round PCR products were diluted 1/10 and used in a second PCR amplification with two Amb a X peptidic sequence-derived primers (see Table 4).

**Table 4: primers combinations used in RACE experiments**

| **1st round** | | **2nd round** | |
|---|---|---|---|
| **Forward primer** | **Reverse Primer** | **Forward primer** | **Reverse Primer** |
| STA390 | Universal primer | STA388 | STA208 |
| STA390 | Universal primer | STA400 | STA208 |
| STA400 | Universal primer | STA390 | STA208 |
| STA476 | Universal primer | | |
| STA490 | Universal primer | | |
| STA536 | Universal primer | | |
| STA538 | Universal primer | | |
| Universal primer | STA517 | | |
| STA496 | Universal primer | | |
| STA510 | Universal primer | | |
| STA516 | Universal primer | | |
| Universal primer | STA505 | | |
| Universal primer | STA497 | | |
| Universal primer | STA539 | | |
| Universal primer | STA537 | | |
| Universal primer | STA538 | | |

After RACE experiments, amplified DNA fragments were loaded on 1 % agarose gel stained with ethidium bromide and separates by electrophoresis. Bands of interest were excised and DNA was extracted. Purified DNA fragments were then cloned in pCR4-TOPO vector using the T/A cloning for sequencing kit and TOP10 competent cells.

In order to get missing nucleotide sequences at the 5' and 3' extremities (translated and untranslated regions), a set of overlapping primers was subsequently designed based on sequencing data. These specific primers were used in combination with UPM primer on ragweed pollen cDNA as described above. PCR cycles were performed with annealing temperature between 50-65°C. Amplified DNA fragments were purified and cloned as described above.

### Example 2.2.3: Homology searches

DNA as well as translated sequences were blasted against NCBI database using Blastn, Blastp, Blastx, tBlastn and tBlastx algorithms (nr nucleotide collection and nr protein database, restricted to green plants) to search for sequence homologies.

### Example 2.3: Results and interpretation

### Example 2.3.1: Cloning by RACE approach

Peptide sequences obtained by N-terminal sequencing and E2D-PAGE-LC-MS/MS were used to design a set of non degenerated primers. Codons were chosen upon frequency tables. RT-PCR cloning of Amb a X was performed using RACE method on total RNA extracted from ragweed pollen. Two successive amplifications were done as described in methods. Amplified DNA fragments were submitted to T/A cloning and sequencing. Independent clones overlapping fragments were found to contain sequences coding both N-terminal peptide and internal peptides.

Partial sequence information was subsequently used to design overlapping primers. A second round of RACE experiments was then performed to obtain missing sequences in the 5' and 3' regions. As for internal regions, amplified fragments were cloned and submitted to DNA sequencing. 5', internal and 3' sequences were assembled to obtain complete Amb a X sequence.

### Example 2.3.2: Sequence homologies

Based on the different Amb a X fragment, the following consensus sequence was established for the pre-pro-form of Amb a X:

MEINKLVCFSFSLVLILGLVESFHYHERELESEEGFMGMYDRWREQHNIEMRSPE
RFNVFKYNVRRIHESNKMDKPYKLKVNEFADMTNLEFVNTYANSKISHFQALRGSAPGSI
DTDPNKDFIYANVTKIPDKVDWREKNAVTDVKGQGGCGSCWAFAAVVALEGINAIRTGK
LVKFSEQQLVDCDMTNAGCDGGLMEPAFTYVIKHGGIAPEASYPYVGKRETCDKAKIKD
VLKIDGRQNVPGLDEEALRKAVAHQPVATGIQLSGHGLQFYSEGVYTGDCGTEPNHGV
GIVGYGENEKGIKFWTVKNSWGPTWGEKGYIHLQRGARKEGLCGVAMHSSFPIMNDPN
PPKDDPNGPKDDPDAPKDPKFKTTQRLQGIRTKLLEL (SEQ ID NO: 28).

DNA sequences as well as translated sequences were blasted against NCBI database to search for sequence homologies using Blastn, Blastp, Blastx, tBlastn and tBlastx algorithms (nr nucleotide collection and nr protein database, restricted to green plants). At both levels, Amb a X fragments exhibited strong homologies with cysteine proteases.

The position of the initiator methionine was determined by sequence homologies with other cysteine proteases and is corroborated by the presence of a Kozak-like sequence (Ribosome Binding Site) around the proximal ATG codon (ACAATAATGG, SEQ ID NO: 27). Boundaries are referred relatively to this methionine.

Amb a X sequence (SEQ ID NO: 28) corresponds to a pre-pro-protein encompassing a predicted signal sequence (aa 1-22) and a pro-region (aa 23-108) which contains an 129 Cathepsin propeptide inhibitor homologous domain (aa 40-92). The catalytic triad (C₁₅₅, H₂₈₉, N₃₁₀ of SEQ ID NO: 28) as well as 6 cysteines, putatively engaged in disulfide bonds (C₁₅₂-C₁₉₃; C₁₈₆-C₂₂₆; C₂₈₃-C₃₃₄ of SEQ ID NO: 28), are conserved. Futhermore, N-Glycosylation (N₁₂₇ of SEQ ID NO: 28) is confirmed by MS analyses.

The Amb a X sequence was corroborated by nano LC-MS/MS and MALDI MS analyses of E2D-PAGE spots as well as extended Edman sequencing.

### Example 2.3.3: Polymorphism

A preliminary analysis was done at the nucleotide and amino acid levels to characterize Amb a X polymorphisms. Multiple silent and missense nucleotide variations was observed in DNA sequences. V/I₂₀₅ and A/V₂₁₂ of SEQ ID NO: 28 were confirmed from MS spectra. The high number of single nucleotide variations and amino-acid changes is evocative of Amb a X isoforms/variants.

## Claims

1. An isolated polypeptide comprising:
a) the sequence SEQ ID NO: 1 (Amb a X), or
b) a sequence having at least 57% identity with sequence SEQ ID NO: 1 and which has a same biological activity as the polypeptide of sequence SEQ ID NO: 1, or
c) a variant of the sequence defined in a) or b) which exhibits reduced allergenicity or reduced enzymatic activity as compared with the sequence defined in a) or b), or
d) a derivative of the sequence defined in a), b) or c) which has been modified by thermal, chemical or physical treatment, or
e) a fragment of the sequence defined in a), b) or c), said fragment comprising at least 250 contiguous amino acids of the sequence defined in a), b) or c), or being an epitopic fragment of the sequence defined in a), b) or c).

2. The isolated polypeptide according to claim 1, wherein said polypeptide comprises a sequence having at least 67% identity with sequence SEQ ID NO: 1, and wherein:
(i) the molecular weight of said polypeptide differs by no more than 10% from the molecular weight of a polypeptide consisting of SEQ ID NO: 1, and
(ii) said polypeptide has the same biological activity as the polypeptide of sequence SEQ ID NO: 1.

3. The isolated polypeptide according to claim 1 or 2, wherein said polypeptide comprises a sequence having at least 85% identity with sequence SEQ ID NO: 1.

4. An isolated nucleic acid which comprises a sequence encoding a polypeptide as defined in any one of claims 1 to 3.

5. A primer or a probe hybridizable to the isolated nucleic acid as defined in claim 4 under standard hybridization conditions.

6. A vector comprising a nucleic acid sequence encoding a polypeptide as defined in any one of claims 1 to 3 which is operatively associated with expression control sequences.

7. A host cell containing a nucleic acid sequence encoding a polypeptide as defined in any one of claims 1 to 3 or a vector as defined in claim 6.

8. An in vitro method for preparing a polypeptide as defined in any one of claims 1 to 3, said method comprising:
a) culturing a host cell as defined in claim 7 under conditions suitable to obtain expression of a polypeptide as defined in one of claims 1 to 3; and
b) recovering the expressed polypeptide.

9. An in vivo method for preparing a polypeptide as defined in any one of claims 1 to 3, said method comprising:
a) culturing a prokaryote or eukaryote organism transformed by a nucleic acid as defined in claim 4 or a vector as defined in claim 6 under conditions and for a sufficient length of time to enable expression of said polypeptide, and
b) isolating polypeptides produced from the transformed organisms.

10. An isolated antibody which binds specifically to a polypeptide as defined in any one of claims 1 to 3.

11. A pharmaceutical composition comprising a polypeptide as defined in any one of claims 1 to 3, or an antibody as defined in claim 10, and a pharmaceutically acceptable carrier.

12. The polypeptide as defined in any one of claims 1 to 3, or the antibody as defined in claim 10, for use as a medicament.

13. The polypeptide as defined in any one of claims 1 to 3, or the antibody as defined in claim 10, for use for preventing or treating an allergic reaction to ragweed pollen.

14. The polypeptide as defined in any one of claims 1 to 3 for use for detecting an allergy or sensitivity to ragweed pollen.

15. An in vitro method of diagnosing an allergy or sensitivity to ragweed pollen in an individual, said method comprising the steps consisting of:
a) incubating a polypeptide as defined in any one of claims 1 to 3 with a biological sample of an individual;
b) detecting the presence or absence of immune complexes between said polypeptide and IgEs from said biological sample of the individual;
wherein the presence of immune complexes between said polypeptide and IgEs from said biological sample of the individual indicates that the individual is sensitized or allergic to ragweed pollen.

16. A kit for the diagnosis of an allergy comprising:
a) a polypeptide as defined in any one of claims 1 to 3; and
b) a lancet; and/or
c) instructions for use.

17. An in vitro method for detecting a polypeptide as defined in any one of claims 1 to 3 in a sample, said method comprising the steps consisting of:
a) incubating a sample with an antibody as defined in claim 10;
b) detecting the presence or absence of immune complexes comprising said antibody;
wherein the presence of immune complexes comprising said antibody is indicative of the presence of a polypeptide as defined in any one of claims 1 to 3 in said sample.

18. An in vitro method for quantifying a polypeptide according to the invention in a sample, said method comprising the steps consisting of:
a) providing a known amount of the polypeptide as defined in claim 1, optionally labelled, as a calibration standard,
b) degrading the sample containing the polypeptide to be quantified to obtain a mixture of polypeptides, optionally labelled,
wherein at least the polypeptides in the degraded sample or in the calibration standard are labelled, and if both polypeptides are labelled, the labelling agent used for the polypeptides in the calibration standard is different from the labelling agent used for the polypeptides in the degraded sample,
c) quantifying the absolute amount of the polypeptide according to the invention in the sample by correlating the amount of the polypeptide in the calibration standard with the amount of the corresponding polypeptide in the degraded sample by mass analysis.


SEQUENCE LISTING

<110>  STALLERGENES S.A.

<120>  NOVEL ALLERGEN FROM RAGWEED POLLEN AND USES THEREOF

<130>  BET 12P0066

<160>  41

<170>  PatentIn version 3.4

<210>  1
<211>  278
<212>  PRT
<213>  Ambrosia artemisiifolia

<400>  1

Gly Ser Ala Pro Gly Ser Ile Asp Thr Asp Pro Asn Lys Asp Phe Ile
1               5                   10                  15

Tyr Ala Asn Val Thr Lys Ile Pro Asp Lys Val Asp Trp Arg Glu Lys
            20                  25                  30

Asn Ala Val Thr Asp Val Lys Gly Gln Gly Gly Cys Gly Ser Cys Trp
        35                  40                  45

Ala Phe Ala Ala Val Val Ala Leu Glu Gly Ile Asn Ala Ile Arg Thr
    50                  55                  60

Gly Lys Leu Val Lys Phe Ser Glu Gln Gln Leu Val Asp Cys Asp Met
65                  70                  75                  80

Thr Asn Ala Gly Cys Asp Gly Gly Leu Met Glu Pro Ala Phe Thr Tyr
                85                  90                  95

Val Ile Lys His Gly Gly Ile Ala Pro Glu Ala Ser Tyr Pro Tyr Val
            100                 105                 110

Gly Lys Arg Glu Thr Cys Asp Lys Ala Lys Ile Lys Asp Val Leu Lys
        115                 120                 125

Ile Asp Gly Arg Gln Asn Val Pro Gly Leu Asp Glu Glu Ala Leu Arg
    130                 135                 140

Lys Ala Val Ala His Gln Pro Val Ala Thr Gly Ile Gln Leu Ser Gly
145                 150                 155                 160

His Gly Leu Gln Phe Tyr Ser Glu Gly Val Tyr Thr Gly Asp Cys Gly
                165                 170                 175

Thr Glu Pro Asn His Gly Val Gly Ile Val Gly Tyr Gly Glu Asn Glu
            180                 185                 190

Lys Gly Ile Lys Phe Trp Thr Val Lys Asn Ser Trp Gly Pro Thr Trp
        195                 200                 205

Gly Glu Lys Gly Tyr Ile His Leu Gln Arg Gly Ala Arg Lys Glu Gly
    210                 215                 220

Leu Cys Gly Val Ala Met His Ser Ser Phe Pro Ile Met Asn Asp Pro
225                 230                 235                 240

Asn Pro Pro Lys Asp Asp Pro Asn Gly Pro Lys Asp Asp Pro Asp Ala
                245                 250                 255

Pro Lys Asp Pro Lys Phe Lys Thr Thr Gln Arg Leu Gln Gly Ile Arg
            260                 265                 270

Thr Lys Leu Leu Glu Leu
        275

<210>  2
<211>  21
<212>  DNA
<213>  Artificial

<220>
<223>  oligonucleotide

<400>  2
aacgccgtta ccgatgttaa a                                                21

<210>  3
<211>  21
<212>  DNA
<213>  Artificial

<220>
<223>  oligonucleotide

<400>  3
ccagaagcca gctacccata c                                                21

<210>  4
<211>  24
<212>  DNA
<213>  Artificial

<220>
<223>  oligonucleotide

<400>  4
accccaggtt ggaccccaag agtt                                             24

<210>  5
<211>  20
<212>  DNA

<213>  Artificial

<220>
<223>  oligonucleotide

<400>  5
tcttacccat acgttggtaa                                                    20


<210>  6
<211>  35
<212>  DNA
<213>  Artificial

<220>
<223>  oligonucleotide

<400>  6
catggtggta tcgccccaga agccagctac ccata                                   35


<210>  7
<211>  35
<212>  DNA
<213>  Artificial

<220>
<223>  oligonucleotide

<400>  7
catggtggtt tggccccaga agcctcttac ccata                                   35


<210>  8
<211>  44
<212>  DNA
<213>  Artificial

<220>
<223>  oligonucleotide

<400>  8
catggtggtt tggccccaga agccagctac ccatacgttg gtaa                         44


<210>  9
<211>  35
<212>  DNA
<213>  Artificial

<220>
<223>  oligonucleotide

<400>  9
catggtggtc tcgccccaga agcctcttac ccata                                   35


<210>  10
<211>  38
<212>  DNA
<213>  Artificial

<220>
<223>  oligonucleotide

<400>  10
caaaacgttc caggtatcga tgaagaagcc atcagaaa                              38


<210>  11
<211>  38
<212>  DNA
<213>  Artificial

<220>
<223>  oligonucleotide

<400>  11
caaaacgttc caggtatcga tgaagaagcc ctcagaaa                              38


<210>  12
<211>  38
<212>  DNA
<213>  Artificial

<220>
<223>  oligonucleotide

<400>  12
caaaacgttc caggtttgga tgaagaagcc atcagaaa                              38


<210>  13
<211>  38
<212>  DNA
<213>  Artificial

<220>
<223>  oligonucleotide

<400>  13
caaaacgttc caggtctcga tgaagaagcc atcagaaa                              38


<210>  14
<211>  38
<212>  DNA
<213>  Artificial

<220>
<223>  oligonucleotide

<400>  14
caaaacgttc caggtctcga tgaagaagcc ttgagaaa                              38


<210>  15
<211>  33
<212>  DNA
<213>  Artificial

<220>
<223>  oligonucleotide

<400>  15
gataccgacc ctaataaaga tttcatatat gca                                   33


<210>  16

<211>  33
<212>  DNA
<213>  Artificial

<220>
<223>  oligonucleotide

<400>  16
ggcaaaagag aaacctgcga caaagcaaag att                                    33


<210>  17
<211>  30
<212>  DNA
<213>  Artificial

<220>
<223>  oligonucleotide

<400>  17
ggacttgacg aagaagcact aaggaaggca                                        30


<210>  18
<211>  30
<212>  DNA
<213>  Artificial

<220>
<223>  oligonucleotide

<400>  18
tgccttcctt agtgcttctt cgtcaagtcc                                        30


<210>  19
<211>  30
<212>  DNA
<213>  Artificial

<220>
<223>  oligonucleotide

<400>  19
tcccacaatt ccaacaccat gattcggctc                                        30


<210>  20
<211>  33
<212>  DNA
<213>  Artificial

<220>
<223>  oligonucleotide

<400>  20
gtcactgatg tcaagggtca aggcggatgt gga                                    33


<210>  21
<211>  21
<212>  DNA
<213>  Artificial

<220>

<223>  oligonucleotide

<400>  21
gtaaaatttt ccgaacaaca a                                                21


<210>  22
<211>  21
<212>  DNA
<213>  Artificial

<220>
<223>  oligonucleotide

<400>  22
ttgttgttcg gaaaatttta c                                                21


<210>  23
<211>  42
<212>  DNA
<213>  Artificial

<220>
<223>  oligonucleotide

<400>  23
tcagcacctg gctcgattga taccgaccct aataaagatt tc                         42


<210>  24
<211>  42
<212>  DNA
<213>  Artificial

<220>
<223>  oligonucleotide

<400>  24
gaaatcttta ttagggtcgg tatcaatcga gccaggtgct ga                         42


<210>  25
<211>  42
<212>  DNA
<213>  Artificial

<220>
<223>  oligonucleotide

<400>  25
tcagcacctg gctcgatcga taccgaccct aataaagatt tc                         42


<210>  26
<211>  42
<212>  DNA
<213>  Artificial

<220>
<223>  oligonucleotide

<400>  26
gaaatcttta ttagggtcgg tatcgatcga gccaggtgct ga                         42

<210>  27
<211>  10
<212>  DNA
<213>  Artificial

<220>
<223>  cDNA

<400>  27
acaataatgg                                                            10


<210>  28
<211>  386
<212>  PRT
<213>  Ambrosia artemisiifolia


<220>
<221>  SIGNAL
<222>  (1)..(22)

<220>
<221>  PROPEP
<222>  (23)..(386)

<220>
<221>  PEPTIDE
<222>  (109)..(386)

<400>  28

Met Glu Ile Asn Lys Leu Val Cys Phe Ser Phe Ser Leu Val Leu Ile
1               5                   10                  15


Leu Gly Leu Val Glu Ser Phe His Tyr His Glu Arg Glu Leu Glu Ser
            20                  25                  30


Glu Glu Gly Phe Met Gly Met Tyr Asp Arg Trp Arg Glu Gln His Asn
        35                  40                  45


Ile Glu Met Arg Ser Pro Glu Arg Phe Asn Val Phe Lys Tyr Asn Val
    50                  55                  60


Arg Arg Ile His Glu Ser Asn Lys Met Asp Lys Pro Tyr Lys Leu Lys
65                  70                  75                  80


Val Asn Glu Phe Ala Asp Met Thr Asn Leu Glu Phe Val Asn Thr Tyr
                85                  90                  95


Ala Asn Ser Lys Ile Ser His Phe Gln Ala Leu Arg Gly Ser Ala Pro
            100                 105                 110


Gly Ser Ile Asp Thr Asp Pro Asn Lys Asp Phe Ile Tyr Ala Asn Val
        115                 120                 125

Thr Lys Ile Pro Asp Lys Val Asp Trp Arg Glu Lys Asn Ala Val Thr
    130                 135                 140

Asp Val Lys Gly Gln Gly Gly Cys Gly Ser Cys Trp Ala Phe Ala Ala
145                 150                 155                 160

Val Val Ala Leu Glu Gly Ile Asn Ala Ile Arg Thr Gly Lys Leu Val
                165                 170                 175

Lys Phe Ser Glu Gln Gln Leu Val Asp Cys Asp Met Thr Asn Ala Gly
            180                 185                 190

Cys Asp Gly Gly Leu Met Glu Pro Ala Phe Thr Tyr Val Ile Lys His
        195                 200                 205

Gly Gly Ile Ala Pro Glu Ala Ser Tyr Pro Tyr Val Gly Lys Arg Glu
    210                 215                 220

Thr Cys Asp Lys Ala Lys Ile Lys Asp Val Leu Lys Ile Asp Gly Arg
225                 230                 235                 240

Gln Asn Val Pro Gly Leu Asp Glu Glu Ala Leu Arg Lys Ala Val Ala
                245                 250                 255

His Gln Pro Val Ala Thr Gly Ile Gln Leu Ser Gly His Gly Leu Gln
            260                 265                 270

Phe Tyr Ser Glu Gly Val Tyr Thr Gly Asp Cys Gly Thr Glu Pro Asn
        275                 280                 285

His Gly Val Gly Ile Val Gly Tyr Gly Glu Asn Glu Lys Gly Ile Lys
    290                 295                 300

Phe Trp Thr Val Lys Asn Ser Trp Gly Pro Thr Trp Gly Glu Lys Gly
305                 310                 315                 320

Tyr Ile His Leu Gln Arg Gly Ala Arg Lys Glu Gly Leu Cys Gly Val
                325                 330                 335

Ala Met His Ser Ser Phe Pro Ile Met Asn Asp Pro Asn Pro Pro Lys
            340                 345                 350

Asp Asp Pro Asn Gly Pro Lys Asp Asp Pro Asp Ala Pro Lys Asp Pro
        355                 360                 365

Lys Phe Lys Thr Thr Gln Arg Leu Gln Gly Ile Arg Thr Lys Leu Leu
    370                 375                 380

Glu Leu

385

<210>  29
<211>  1161
<212>  DNA
<213>  Ambrosia artemisiifolia

<400>  29
atggaaatca acaagttagt ttgtttttca ttttctttgg ttttgatttt aggacttgta      60

gagagcttcc attaccatga gagagagctc gaatcggagg aggggtttat ggggatgtat     120

gatagatgga gggagcaaca caatatcgaa atgagaagcc cggaacggtt caatgtgttc     180

aagtacaatg ttaggcgcat tcacgaatcg aataagatgg acaagccgta taagttgaag     240

gtgaacgagt ttgctgacat gactaacctt gagttcgtta acacgtatgc taactcgaag     300

attagccatt ttcaagccct ccgaggatca gcacctggct cgattgatac cgaccctaat     360

aaagatttca tatatgcaaa tgtcactaaa atcccagata aggtcgattg gagggagaaa     420

aacgctgtca ctgatgtcaa gggtcaaggc ggatgtggaa gttgttgggc gtttgccgct     480

gtggttgcac tggaaggaat aaacgcgatc agaaccggga agctggtaaa attttccgaa     540

caacaacttg tcgattgtga catgacgaac gcaggatgcg acggagggct aatggaacct     600

gcattcacat acgtcataaa gcatggaggt atagctccag aagcgagcta cccttacgta     660

ggcaaaagag aaacctgcga caaagcaaag attaaagatg tgttgaagat cgatggtaga     720

caaaatgtgc ctggacttga cgaagaagca ctaaggaagg cagttgcaca ccagcctgta     780

gctaccggta tacaacttag cggccatggt ttgcagttct attccgaggg tgtatatacc     840

ggagattgtg gtacagagcc gaatcatggt gttggaattg tgggatacgg tgagaatgaa     900

aaggggatta aattctggac cgtgaagaac tcatggggac caacatgggg agagaaggga     960

tacatacatt tacaacgcgg agctaggaaa gagggactat gcggagtagc aatgcattct    1020

tcttttccta ttatgaacga cccaaaccca cctaaagacg accccaatgg acctaaagac    1080

gaccctgatg cacctaagga ccccaaattt aaaacgactc agaggttgca agggataagg    1140

actaaattgt tggagttgtg a                                              1161

<210>  30
<211>  15
<212>  PRT
<213>  Ambrosia artemisiifolia

<400>  30

Gly Ser Ala Pro Gly Ser Ile Asp Thr Asp Pro Asn Lys Asp Phe
1               5                   10                  15

<210>  31
<211>  25
<212>  PRT
<213>  Ambrosia artemisiifolia

<400>  31

Gly Ser Ala Pro Gly Ser Ile Asp Thr Asp Pro Asn Lys Asp Phe Ile
1               5                   10                  15

Tyr Ala Asn Val Thr Lys Ile Pro Asp
            20                  25

<210>  32
<211>  15
<212>  PRT
<213>  Ambrosia artemisiifolia

<400>  32

Val Thr Asp Val Lys Gly Gln Gly Gly Cys Gly Ser Cys Trp Ala
1               5                   10                  15

<210>  33
<211>  15
<212>  PRT
<213>  Ambrosia artemisiifolia

<400>  33

Phe Ala Ala Val Val Ala Leu Glu Gly Ile Asn Ala Ile Arg Thr
1               5                   10                  15

<210>  34
<211>  15
<212>  PRT
<213>  Ambrosia artemisiifolia

<400>  34

Leu Glu Gly Ile Asn Ala Ile Arg Thr Gly Lys Leu Val Lys Phe
1               5                   10                  15

<210>  35
<211>  15
<212>  PRT
<213>  Ambrosia artemisiifolia

<400>  35

Ala Phe Thr Tyr Val Ile Lys His Gly Gly Ile Ala Pro Glu Ala
1               5                   10                  15

<210>  36
<211>  15
<212>  PRT
<213>  Ambrosia artemisiifolia

<400>  36

Gly Gly Leu Met Glu Pro Ala Phe Thr Tyr Val Ile Lys His Gly
1               5                   10                  15

<210>  37
<211>  15
<212>  PRT
<213>  Ambrosia artemisiifolia

<400>  37

Lys Asp Val Leu Lys Ile Asp Gly Arg Gln Asn Val Pro Gly Leu
1               5                   10                  15


<210>  38
<211>  15
<212>  PRT
<213>  Ambrosia artemisiifolia

<400>  38

Ser Phe Pro Ile Met Asn Asp Pro Asn Pro Pro Lys Asp Asp Pro
1               5                   10                  15


<210>  39
<211>  15
<212>  PRT
<213>  Ambrosia artemisiifolia

<400>  39

Pro Asn Lys Asp Phe Ile Tyr Ala Asn Val Thr Lys Ile Pro Asp
1               5                   10                  15


<210>  40
<211>  15
<212>  PRT
<213>  Ambrosia artemisiifolia

<400>  40

Gly Val Gly Ile Val Gly Tyr Gly Glu Asn Glu Lys Gly Ile Lys
1               5                   10                  15


<210>  41
<211>  15
<212>  PRT
<213>  Ambrosia artemisiifolia

<400>  41

Glu Lys Gly Ile Lys Phe Trp Thr Val Lys Asn Ser Trp Gly Pro
1               5                   10                  15